# EUROPEAN PATENT APPLICATION

(11) **EP 4 400 590 A1**
(43) Date of publication of application: **17.07.2024**
(21) Application number: 22867747.2
(22) Date of filing: 08.09.2022
(51) Int. Cl.: C12N 15/63, C07K 14/705, G01N 33/574, A61K 48/00, A61P 35/00

(54) **PLASMID PLATFORM FOR STABLE EXPRESSION AND DELIVERY OF BIOMOLECULES**

(30) Priority: 08.09.2021 KR 20210119735
(71) Applicant: Kkoomlab Inc., Yongin-si, Gyeonggi-do 16942 (KR)
(72) Inventor: JANG, Kang Won, Yongin-si, Gyeonggi-do 16811 (KR)
(74) Representative: Gulde & Partner
(86) International application number: PCT/KR2022/013574
(87) International publication number: WO 2023/038479

(57) **Abstract**

A plasmid platform according to the present invention comprises a nucleic acid sequence encoding a modified protein from which an intracellular domain, an extracellular domain, or a combination thereof of lysosome-associated membrane glycoprotein 2B (LAMP-2B) has been removed, and is based on the discovery that removing a specific domain of LAMP-2B significantly stabilizes the expression of intended biomolecules and significantly increases the efficiency with which intended biomolecules are delivered. The plasmid platform is expected to provide high technical utility in the expression and delivery of proteins of interest, particularly expression and delivery using exosomes, and may be expanded to various associated technical fields or uses.

## Description

### [Technical Field]

The present invention relates to a plasmid platform for the stable expression and delivery of a biomolecule.

### [Background Art]

Exosomes are produced in the budding of late endosomes and known to fuse with a plasma membrane before being released into the extracellular space. Exosomes are 40 to 200-nm vesicles composed of a lipid bilayer rich in phosphocholine, cholesterol, and ceramide, secreted from almost all types of cells, and stably present in all types of body fluids such as blood, lymph, and sweat. In addition, exosomes have the advantages of a long circulation time in the body and reaching the inside of organs due to their small size and weak negative charge. Exosomes can also deliver hydrophilic or hydrophobic drugs by evading phagocytosis and can pass through the vascular endothelium to target cells. Exosomes are known to have targeted effects on specific cells due to a specific surface protein such as tetraspanin. It has been reported that the encapsulation of exosomes increases the stability and bioavailability of curcumin *in vitro* and *in vivo,* and increases anti-inflammatory activity. In another study, it has been reported that exosomes deliver doxorubicin to the brain across the blood-brain barrier, deliver siRNA to cells, and effectively reduce the level of RAD 51 protein, which is a powerful target for cancer treatment.

### [Disclosure]

### [Technical Problem]

The present invention is directed to providing a plasmid platform for the stable expression and delivery of a biomolecule, which includes a nucleic acid sequence encoding a modified protein from which the intracellular domain or extracellular domain of lysosome-associated membrane glycoprotein 2B (LAMP-2B), or a combination thereof has been removed.

The present invention also directed to providing a recombinant plasmid for the stable expression and delivery of a biomolecule, which further includes a sequence encoding a biomolecule to be expressed and delivered in the plasmid platform.

The present invention also directed to providing an exosome for the stable expression and delivery of a biomolecule, which includes the recombinant plasmid.

The present invention also directed to providing a composition for diagnosing cancer, which includes the exosomes, and here, the biomolecule is a peptide specifically binding to a protein specifically expressed on the surface of cancer cells

However, technical problems to be solved in the present invention are not limited to the above-described technical problems, and other technical problems which are not described herein will be fully understood by those of ordinary skill in the art from the following descriptions.

### [Technical Solution]

One aspect of the present invention provides a plasmid platform for the stable expression and delivery of a biomolecule, which includes a nucleic acid sequence encoding a modified protein from which the intracellular domain or extracellular domain of lysosome-associated membrane glycoprotein 2B (LAMP-2B), or a combination thereof has been removed.

The plasmid platform is based on the finding that, when the intracellular or extracellular domain of conventionally known LAMP-2B, or both are removed, the stability of the expression of a target protein to be expressed intracellularly or extracellularly, together with LAMP-2B, is significantly increased, and the deliverability to target cells is also significantly increased.

The LAMP-2B relates to a conventionally known amino acid sequence, and a nucleic acid sequence encoding the same, and refers to a nucleic acid sequence to be loaded in a plasmid platform. According to a specific embodiment, it may be the sequence assigned Accession No. NM_013995.2, but the present invention is not particularly limited thereto.

The intracellular domain and the extracellular domain may refer to a region expressed inside the phospholipid bilayer of a cell and a region expressed outside the phospholipid bilayer thereof upon the expression of LAMP-2B on cells, respectively. Considering the case where LAMP-2B is expressed on exosomes, preferably, the intracellular domain refers to the region expressed inside the phospholipid bilayer of an exosome, and the extracellular domain refers to the region expressed outside the phospholipid bilayer of an exosome.

The modified protein is characterized by the removal of the intracellular domain or extracellular domain of LAMP-2B, or a combination thereof, and in order to stabilize the expression of a biomolecule (or a target protein) to be expressed in an extracellular region (or the external region of the phospholipid bilayer), and a biomolecule (or an active protein) to be expressed in an intracellular region (or the internal region of the phospholipid bilayer), preferably, the intracellular domain may be removed. In this sense, the plasmid platform may contain a nucleic acid sequence that encodes a protein from which the intracellular domain of LAMP-2B has been removed.

In addition, both the intracellular domain and the extracellular domain are preferably removed from the modified protein not only to stabilize the expression of a biomolecule to be expressed in the extracellular region and a biomolecule to be expressed in the intracellular region but also to stably deliver the biomolecules to desired cells. In this sense, the plasmid platform may have neither the intracellular domain nor the extracellular domain of LAMP-2B.

According to one embodiment, the nucleic acid sequence may have a nucleic acid sequence of any one of SEQ ID NOs: 2 to 4, and preferably has the nucleic acid sequence of SEQ ID NO: 2 or 4 to stabilize the expression of the biomolecule to be expressed in the intracellular region. More preferably, the nucleic acid sequence has the nucleic acid sequence of SEQ ID NO: 4 not only to stabilize the expression of the biomolecule to be expressed in the intracellular region but also to stably deliver the biomolecule to desired cells.

The plasmid platform may further include a nucleic acid sequence encoding a glycosylated region to stabilize the expression and delivery of a biomolecule to be expressed by being loaded in the plasmid platform. More specifically, this may be to stabilize the expression and delivery of a biomolecule in both the intracellular region and the extracellular region, but the present invention is not limited thereto.

The glycosylated region may be freely introduced using a known means that can induce glycosylation in a protein expression structure and may be formed such that the nucleic acid sequence encoding the glycosylated region is included in the plasmid platform in terms of application to the plasmid platform. For example, at least one method may be selected from a method of including a nucleic acid sequence encoding a GNSTM motif in the plasmid platform; a method of including a sequence encoding an amino acid-specific sequence that can induce N-linked glycosylation NXS (X can be any amino acid except proline), NXT (X can be any amino acid except proline), or NXC (X can be any amino acid except proline); a method of further including a sequence encoding 1 to 5 amino acids in the C-terminal part of the N-glycosylation amino acid sequence; a method of including a sequence encoding glycine in the N-terminal part of a sequence including 1 to 5 amino acids in the C-terminal part of the N-glycosylation amino acid sequence; a method of including a sequence encoding an N-linked glycosylation motif (CD9 SEL: small extracellular loop domain, CD63 LEL: large extracellular loop domain, etc.) in tetraspanin; and a method of including serine or threonine, which are amino acids inducing O-glycosylation. In addition, to maximize both the stable expression and delivery efficiency of a biomolecule, preferably, the method of including a nucleic acid sequence encoding a GNSTM motif in the plasma platform is selected to introduce glycosylation, but the present invention is not particularly limited thereto.

The location of the nucleic acid sequence that encodes the glycosylated region is not particularly limited as long as the nucleic acid sequence can be included in the plasmid platform, but, for example, the nucleic acid sequence that encodes the glycosylated region may be included so as to be located in a direction of an extracellular region (or the external region of the phospholipid bilayer) based on the nucleic acid sequence encoding the modified protein. As a more specific example, a sequence encoding a biomolecule (or a target protein) may be located in a direction of the extracellular region of the nucleic acid sequence encoding the modified protein, and the nucleic acid sequence encoding the glycosylated region may be located upstream (or an extracellular region direction) or downstream (or an intracellular region direction) of the sequence encoding the biomolecule, but the present invention is not particularly limited thereto. According to a preferred embodiment, the nucleic acid sequence encoding the glycosylated region may be located in an extracellular region direction based on the nucleic acid sequence encoding the modified protein.

The nucleic acid sequence encoding the glycosylated region may include a nucleic acid sequence of any one of SEQ ID NOs: 11 to 13, and preferably includes the nucleic acid sequence of SEQ ID NO: 11 to maximize both the stable expression and delivery efficiency of a biomolecule.

The plasmid platform may have a coding sequence to have both the glycosylation region and the modified protein form, and the detailed description for each is as described above. According to a preferred embodiment, the plasmid platform may include both a sequence encoding a GNSTM motif and a sequence encoding a modified protein from which both the extracellular domain and the intracellular domain of LAMP-2B are removed. As a more specific example, the sequence encoding the GNSTM motif may be included to be located upstream (or an extracellular region direction) of the sequence encoding the modified protein. As an additional example, the plasmid platform may include the nucleic acid sequence of SEQ ID NO: 4 and the nucleic acid sequence of SEQ ID NO: 11.

Another aspect of the present invention provides a recombinant plasmid for the stable expression and delivery of a biomolecule, which further includes a nucleic acid sequence encoding a biomolecule to be expressed and delivered in the above-described plasma platform.

The location of the nucleic acid sequence encoding the biomolecule is not particularly limited, as long as the nucleic acid sequence is located in the above-described plasmid platform to successfully express a desired biomolecule. As a specific example, the nucleic acid sequence may be located i) between the nucleic acid sequence encoding a glycosylated region and the nucleic acid sequence encoding a modified protein, ii) in an intracellular region direction based on the nucleic acid sequence encoding a modified protein, or iii) at both locations.

When the nucleic acid sequence encoding the biomolecule is located in an extracellular direction based on the nucleic acid sequence encoding the modified protein, it may be to express or deliver the biomolecule extracellularly (or the external region of the phospholipid bilayer), and when being located in an intracellular direction based on the nucleic acid sequence encoding the modified protein, it may be to express or deliver the biomolecule intracellularly (or the internal region of the phospholipid bilayer), but the present invention is not particularly limited thereto.

The biomolecule is not particularly limited, as long as it is expressed using the plasmid platform, and may be, specifically, at least one selected from the group consisting of a nucleic acid molecule, an aptamer, a peptide, a protein, a glycoprotein, a lipoprotein, an immunoglobulin, a hormone, a growth factor, a recombinase, and a fluorescent protein.

Still another aspect of the present invention provides an exosome for the stable expression and delivery of a biomolecule, which includes a product expressed from the above-described recombinant plasmid.

The exosome includes a modified protein expressed from the above-described recombinant plasmid and a biomolecule, wherein the biomolecule may be located inside or outside the exosome, or both to be stably expressed, and may be delivered to desired cells.

The biomolecule may include a material that is expressed outside the exosome and specifically binds to the surface of a target cell. In this case, the exosome has increased targetability to a targeted cell to effectively deliver the expressed biomolecule and detect the expressed biomolecule to determine the presence of the targeted cells. Moreover, exosomes may be used for various known uses based on targeting to a target cell without particular limitation. As a specific example, when a biomolecule that can specifically bind to a material specifically expressed on the surface of a cell infected by a specific virus or a cancer cell is expressed outside the exosome, the exosome may specifically bind to a cell infected by a specific virus or a cancer cell, by which the infection of a specific virus or the presence of cancer cells may be determined and the biomolecule included in the exosome may be delivered into the infected cell or cancer cell at the same time.

Yet another aspect of the present invention provides a composition for diagnosing cancer, which includes the above-described exosome, and the biomolecule specifically binds to a protein specifically expressed on the surface of cancer cells.

Yet another aspect of the present invention provides a pharmaceutical composition for preventing or treating cancer, which includes the above-described exosome, and the biomolecule specifically binds to a protein specifically expressed on the surface of cancer cells and includes a therapeutic material to be delivered into cancer cells.

The biomolecule specifically binds to a protein specifically expressed on the surface of cancer cells and is expressed in the external region of the bilayer of the exosome. In this case, as the biomolecule expressed in the external region specifically binds to the surface of a cancer cell, the exosome targets the cancer cell, and can be used for diagnosing cancer based on this targeting.

In addition, the biomolecule may include a therapeutic material to be delivered into cancer cells, and the therapeutic material may be expressed in the internal region of the bilayer of the exosome. In this case, the above-described exosome can deliver the therapeutic material into cancer cells by the interaction with the cancer cells (e.g., surface receptor interaction, membrane fusion, receptor-mediated endocytosis, phagocytosis, or micropinocytosis), resulting in an effect of preventing or treating cancer.

The cancer may be any one selected from the group consisting of carcinomas, including bladder cancer, breast cancer, colon cancer, kidney cancer, liver cancer, lung cancer, ovarian cancer, prostate cancer, pancreatic cancer, stomach cancer, cervical cancer, thyroid cancer, and skin cancer such as squamous cell carcinoma; lymphoid hematopoietic stem tumors, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkett's lymphoma; acute and chronic myeloid leukemia and promyelocytic leukemia; mesenchymal-derived tumors, including fibrosarcoma and rhabdomyomas; other tumors, including melanoma, seminoma, teratocarcinoma, neuroblastoma, and glioma; tumors of the central and peripheral nervous systems, including astrocytoma, neuroblastoma, glioma, and schwannoma; mesenchymal-derived tumors, including fibrosarcoma, rhabdomyosarcoma, and osteosarcoma; and other tumors, including melanoma, xeroderma pigmentosum, keratoacanthoma, seminoma, thyroid follicular carcinoma, and teratocarcinoma, and according to a preferred embodiment, ovarian cancer, but the present invention is not limited thereto.

The composition of the present invention may further include a pharmaceutically acceptable carrier and may be formulated with the carrier. The term "pharmaceutically acceptable carrier" used herein refers to a carrier or diluent that does not irritate an organism and suppress the biological activity and properties of the administered compound. As the pharmaceutically acceptable carrier used in a composition to be prepared as a liquid, saline, sterile water, Ringer's solution, buffered saline, an albumin injection solution, a dextrose solution, a maltodextrin solution, glycerol, ethanol, and a mixture of one or more thereof, which are suitable for sterilization and the living body, may be used, and if needed, other conventional additives such as antioxidant, a buffer, a bacteriostat, etc. may be added. In addition, the composition of the present invention may be formulated into an injectable formulation such as an aqueous solution, a suspension, or an emulsion, a pill, a capsule, a granule, or a tablet by additionally adding a diluent, a dispersant, a surfactant, a binder, and a lubricant.

The composition of the present invention can be applied in any formulation, including the exosome of the present invention as an active ingredient, and may be prepared as an oral or parenteral formulation. The pharmaceutical formulation of the present invention may be prepared in forms suitable for oral, rectal, nasal, topical (including buccal and sublingual), subcutaneous, vaginal, or parenteral (including intranasal, subcutaneous, and intravenous) administration, inhalation, or insufflation.

The composition of the present invention is administered in a pharmaceutically effective amount. The effective dose level may be determined by factors including the type and severity of a patient's disease, drug activity, sensitivity to a drug, administration time, administration route, and excretion rate, treatment duration, a concurrent drug, and other factors well known in the medical field. The pharmaceutical composition of the present invention may be administered as a separate therapeutic agent or administered in combination with another therapeutic agent or may be sequentially or simultaneously administered with a conventional therapeutic agent in a single dose or multiple doses. Considering all the above factors, it is important to administer an amount that can achieve the maximum effect with the minimum amount without side effects, and the amount may be determined easily by those of ordinary skill in the art.

The dosage of the composition of the present invention varies greatly depending on a patient's body weight, age, sex, health condition, diet, administration time, administration method, excretion rate, and severity of a disease, and an appropriate dosage may depend on, for example, the amount of drug accumulated in the patient's body and/or the specific efficacy of a delivery system of the present invention used. Generally, the appropriate dosage may be calculated based on the EC50 measured to be effective in an *in vivo* animal model and *in vitro,* and may be, for example, 0.01 µg to 1 g per kilogram of body weight. The appropriate dosage may be administered once or several times per unit period such as daily, weekly, monthly, or yearly, or may be administered continuously for a long time using an infusion pump. The number of repeated administrations is determined by considering the retention time and drug concentration in the body. Even after treatment, depending on the course of disease treatment, the composition may be administered for relapse.

The composition of the present invention my further contain one or more active ingredients having the same or similar function associated with cancer treatment, or a compound that maintains/increases the solubility and/or absorption of the active ingredient. In addition, selectively, the composition of the present invention may further include a chemotherapeutic, an anti-inflammatory agent, an antiviral agent, and/or an immunomodulatory agent.

In addition, the composition of the present invention may be formulated using a method known in the art to provide rapid, sustained, or delayed release of an active ingredient after being administered to a mammal. The formulation may be in the form of a powder, a granule, a tablet, an emulsion, a syrup, an aerosol, a soft or hard gelatin capsule, a sterile injection solution, or a sterile powder.

All nucleic acid sequences described in the present invention can be modified to a certain extent. It will be easily understood by those of ordinary skill in the art that, as long as a nucleic acid sequence maintaining 70% or more homology by such artificial modification secures the desired activity in the present invention, it is equivalent to that derived from the nucleic acid sequence of the present invention.

The term "homology" refers to the degree of identity with the nucleic acid sequence described above, and for the comparison of homology, the homology between two or more sequences may be compared with the naked eye or by calculation as percentage (%) using a comparative program that can be easily purchased. The nucleic acid sequence of the present invention preferably includes a nucleic acid sequence with 70% or more, more preferably, 80% or more, even more preferably, 90% or more, and most preferably, 95% or more identity therewith.

The term "plasmid" used herein refers to a gene construct containing essential regulatory elements such as a promoter to express a target gene in a suitable host, or may be a form integrated into the genome of a host cell or microorganism.

The "operably linked" used here means that a nucleic acid sequence encoding a target protein is functionally linked with the nucleic acid sequence of a promoter or its variant to perform a general function. The operable linkage with a plasmid may be made using genetic recombination technology well known in the art, and site-specific DNA cleavage and ligation use enzymes generally known in the art.

The "regulatory element" used herein refers to an untranslated nucleic acid sequence that helps or affects the enhancement of the transcription, translation, or expression of a nucleic acid sequence encoding a protein. The plasmid platform of the present invention may include a promoter or its variant as a regulatory element, and expression control sequences that may affect the expression of a protein, e.g., an initiation codon, a termination codon, a polyadenylation signal, an enhancer, and a signal sequence for membrane targeting or secretion.

In addition, when the plasmid of the present invention is a replicable expression plasmid, a replication origin, which is a specific nucleic acid sequence where replication is initiated, may be included.

In addition, the plasmid of the present invention may include a selection marker. A selection marker is used to select a cell or microorganism which is transformed with the plasmid, and markers that impart selectable phenotypes such as drug resistance, auxotrophy, resistance to a cytotoxic agent, or the expression of a surface protein may be used. Transformed individuals can be selected because only cells or organisms expressing a selectable marker survive in an environment treated with a selective agent.

The plasmid of the present invention may have a gene encoding a target biomolecule that is operably linked with the promoter, and particularly, linked downstream of the promoter.

Yet another aspect of the present invention provides a plasmid platform for the stable delivery of a nucleic acid molecule, which includes a nucleic acid sequence that encodes a modified protein from which both the intracellular domain and the extracellular domain of lysosome-associated membrane glycoprotein 2B (LAMP-2B) have been removed; a nucleic acid sequence that encodes a protein specifically binding to a nucleic acid molecule to be delivered; and a nucleic acid sequence encoding a glycosylated region.

The nucleic acid sequence encoding the modified protein may have the nucleic acid sequence of SEQ ID NO: 4.

The nucleic acid sequence encoding the glycosylated region may have at least one nucleic acid sequence selected from the group consisting of SEQ ID NOs: 11 to 13, and preferably, has the nucleic acid sequence of SEQ ID NO: 11 to maximize both the stable expression and delivery efficiency of a biomolecule.

The location of the nucleic acid sequence that encodes the glycosylated region is not particularly limited as long as the nucleic acid sequence can be included in the plasmid platform, but, for example, the nucleic acid sequence that encodes the glycosylated region may be included so as to be located in a direction of an extracellular region (or the external region of the phospholipid bilayer) based on the nucleic acid sequence encoding the modified protein. As a more specific example, a sequence encoding a biomolecule (or a target protein) may be located in a direction of the extracellular region of the nucleic acid sequence encoding the modified protein, and the nucleic acid sequence encoding the glycosylated region may be located upstream (or an extracellular region direction) or downstream (or an intracellular region direction) of the sequence encoding the biomolecule, but the present invention is not particularly limited thereto. According to a preferred embodiment, the nucleic acid sequence encoding the glycosylated region may be located in an extracellular region direction based on the nucleic acid sequence encoding the modified protein.

There is no particular limitation on the location of a nucleic acid sequence that encodes a protein specifically binding to the nucleic acid molecule as long as the nucleic acid sequence can be expressed to specifically bind to a nucleic acid molecule to be delivered and included in the plasma platform, but, for example, the nucleic acid sequence may be included so as to be located in the direction of an intracellular region (or the internal region of the phospholipid bilayer) based on the nucleic acid sequence encoding the modified protein. As a more specific example, a sequence encoding a protein specifically binding to a nucleic acid molecule may be located downstream (or an intracellular region direction) of the nucleic acid sequence encoding the modified protein, and the nucleic acid sequence encoding the glycosylated region may be located upstream (or an extracellular region direction) of a sequence encoding the modified protein, but the present invention is not particularly limited thereto.

The protein specifically binding to the nucleic acid molecule may be at least one selected from the group consisting of a double strand binding motif, a bovine immunodeficiency virus (BIV)-derived binding protein, a Jembrana disease virus (JDV)-derived binding protein, a human immunodeficiency virus (HIV)-derived binding protein, and a variant derived therefrom, and according to a preferred embodiment, a JDV-derived binding protein, but the present invention is not particularly limited thereto.

According to a specific embodiment, the nucleic acid sequence that encodes a protein specifically binding to the nucleic acid molecule may include a nucleic acid sequence having 70% or more homology with at least one sequence selected from the group consisting of SEQ ID NOs: 30 to 34, but the present invention is not particularly limited thereto.

The protein specifically binding to the nucleic acid molecule may include a domain that binds to a specific motif of a nucleic acid molecule to be delivered. According to a specific embodiment, the protein may be a domain specifically binding to a TAR sequence, but the present invention is not limited thereto.

Yet another aspect of the present invention provides a recombinant plasmid for stable delivery of a nucleic acid molecule, which includes the sequence of a nucleic acid molecule to be delivered in the above-described plasmid platform.

The location of the sequence of the nucleic acid molecule in the plasmid is not particularly limited as long as the sequence of the nucleic acid molecule is cleaved by an appropriate splicing process in the state of the plasmid being inserted into cells to maintain stable binding of the nucleic acid molecule to a protein specifically binding thereto. As a preferred embodiment, the sequence of the nucleic acid molecule may be located between the nucleic acid sequence encoding the modified protein and the nucleic acid sequence encoding the protein specifically binding to the nucleic acid molecule.

The nucleic acid molecule may be at least one selected from the group consisting of DNA, RNA, and an aptamer, but the present invention is not limited thereto.

The nucleic acid molecule may include a specific motif to maintain stable binding to the protein specifically binding to the nucleic acid molecule, and as a specific example, include at least one selected from the group consisting of a TAR sequence, a TAR sequence variant, a short double-stranded DNA fragment (preferably, 7 to 27, 9 to 27, 11 to 27, 13 to 27, 7 to 25, 9 to 25, 11 to 25, 13 to 25, or 14 to 25 bp) and a short double-stranded RNA fragment (preferably, 7 to 27, 9 to 27, 11 to 27, 13 to 27, 7 to 25, 9 to 25, 11 to 25, 13 to 25, or 14 to 25 bp), but the present invention is not limited thereto.

Yet another aspect of the present invention provides an exosome for the stable delivery of a nucleic acid molecule, which includes a product expressed from the above-described recombinant plasmid.

Regarding the exosome, the recombinant plasmid may further include a sequence encoding a protein specifically binding to the surface of a target cell by being expressed outside the exosome, which is to increase delivery targetability to a targeted cell and increase the delivery efficiency of a nucleic acid molecule to be delivered.

Regarding the exosome, the protein specifically binding to a nucleic acid molecule may be expressed inside the exosome and bind to the nucleic acid molecule. The protein may be stabilized so as not to degrade the nucleic acid molecule inside the exosome to play a role in ensuring stable delivery to target cells.

Yet another aspect of the present invention provides a composition for the stable delivery of a nucleic acid molecule, which includes the above-described exosome.

Yet another aspect of the present invention provides a composition for RNA interference, which includes an exosome. Here, the nucleic acid molecule has an RNA interference effect.

A nucleic acid molecule having the RNA interference effect may be a non-coding RNA, and preferably, at least one selected from the group consisting of miRNA, shRNA, siRNA, piwi-interacting RNA (piRNA), and long non-coding RNA (lncRNA), but the present invention is not limited thereto.

Yet another aspect of the present invention provides a pharmaceutical composition for preventing or treating cancer, which includes the above-described exosome. Here, the recombinant plasmid further includes a sequence that encodes a protein expressed outside the exosome and specifically binding to the surface of a cancer cell, and the nucleic acid molecule has an effect of preventing or treating cancer.

The composition includes an exosome that is expressed in an external region to specifically bind to a protein specifically expressed on the surface of cancer cells. As one expressed in the external region specifically binds to the surface of a cancer cell, the exosome targets the cancer cell and can be used in preventing or treating cancer based on such targeting.

In addition, the nucleic acid molecule included in the exosome has an effect of preventing or treating cancer. This material may be expressed in the internal region of the bilayer of the exosome and then delivered to a target cancer cell. In this case, the above-described exosome may deliver the nucleic acid molecule into cancer cells by interaction with the cancer cells (e.g., surface receptor interaction, membrane fusion, receptor-mediated endocytosis, phagocytosis, or micropinocytosis), thereby exhibiting a cancer prevention or treatment effect.

The cancer may be any one selected from the group consisting of carcinomas, including bladder cancer, breast cancer, colon cancer, kidney cancer, liver cancer, lung cancer, ovarian cancer, prostate cancer, pancreatic cancer, stomach cancer, cervical cancer, thyroid cancer, and skin cancer such as squamous cell carcinoma; lymphoid hematopoietic stem tumors, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkett's lymphoma; acute and chronic myeloid leukemia and promyelocytic leukemia; mesenchymal-derived tumors, including fibrosarcoma and rhabdomyomas; other tumors, including melanoma, seminoma, teratocarcinoma, neuroblastoma, and glioma; tumors of the central and peripheral nervous systems, including astrocytoma, neuroblastoma, glioma, and schwannoma; mesenchymal-derived tumors, including fibrosarcoma, rhabdomyosarcoma, and osteosarcoma; and other tumors, including melanoma, xeroderma pigmentosum, keratoacanthoma, seminoma, thyroid follicular carcinoma, and teratocarcinoma, and according to a preferred embodiment, ovarian cancer, but the present invention is not limited thereto.

It is obvious to those of ordinary skill in the art that the specific description of the present invention related to the plasmid platform for the stable delivery of the nucleic acid molecule can be interpreted with reference to the specific description of the present invention related to the plasmid platform for stable expression and delivery of the biomolecule.

### [Advantageous Effects]

A plasmid platform of the present invention has a nucleic acid sequence that encodes a modified protein from which the intracellular domain or extracellular domain of lysosome-associated membrane glycoprotein 2B (LAMP-2B), or a combination thereof has been removed, and thus can stably express and deliver a biomolecule.

A recombinant plasmid of the present invention further includes a sequence that encodes a biomolecule to be expressed and delivered in the plasmid platform, and thus can stably express and deliver a biomolecule.

An exosome of the present invention includes the recombinant plasmid, and thus enables the stable expression and delivery of a biomolecule.

A composition for diagnosing cancer of the present invention includes the exosome. Here, the biomolecule is a peptide specifically binding to a protein specifically expressed on the surface of cancer cells, enabling effective diagnosis of cancer.

A pharmaceutical composition for preventing or treating cancer of the present invention includes the exosome. Here, the biomolecule specifically binds to a protein specifically expressed on the surface of cancer cells and includes a therapeutic material to be delivered into cancer cells, and thus can be used in effectively preventing and treating cancer.

However, the present invention is not limited to the above-described effects and should be understood to include all effects that can be inferred from the configuration of the present invention described in the detailed description or claims.

### [Description of Drawings]

FIGS. 1 to 5 schematically illustrate plasmid platforms presented as embodiments of the present invention.
FIG. 6 shows the Western blotting results for LAMP2-GFP, LAMP2-△IC-GFP, LAMP2-△EC-GFP, and LAMP2-△EC/IC-GFP to confirm the expression levels of biomolecules to be expressed.
FIG. 7 shows the graphs quantifying the Western blotting results of FIG. 6.
FIG. 8 shows the results of observing GFP fluorescence levels under a microscope after overexpressing a recombinant plasmid used in the experiment of FIG. 6.
FIG. 9 shows the results of observing GFP fluorescence levels under a microscope after overexpressing LEL-LAMP2-△EC-GFP, and LEL-LAMP2-△EC/IC-GFP.
FIG. 10 shows the Western blotting results for GNSTM-LAMP2-GFP, GNSTM-LAMP2-△IC-GFP, and GNSTM-LAMP2-△EC/IC-GFP to confirm the expression levels of biomolecules to be expressed.
FIG. 11 shows the graphs quantifying the Western blotting results of FIG. 10.
FIG. 12 shows the results of observing GFP fluorescence levels under a microscope after overexpressing a recombinant plasmid used in the experiment of FIG. 10.
FIG. 13 shows the results of observing GFP fluorescence levels under a microscope after overexpressing SEL-LAMP2-△EC-GFP, and SEL-LAMP2-△EC/IC-GFP.
FIG. 14 shows the results of observing GFP fluorescence levels under a microscope after overexpressing GNSTM-LAMP2-△EC/IC-GFP, and Gly-LAMP2-△EC/IC-GFP.
FIG. 15 shows the Western blotting results for GNSTM-LAMP2-GFP, GNSTM-LAMP2-△IC-GFP, GNSTM-LAMP2-△EC/IC-GFP, GNSTM-LAMP2-ECSΔIC-GFP, Gly-LAMP2-ECΔIC-GFP, and LAMP2-EC25△IC-GFP to confirm the expression levels of biomolecules to be expressed.
FIG. 16 shows the results obtained using a microscope to confirm the cancer cell-specific binding ability of exosomes based on the GFP fluorescence levels.
FIGS. 17 to 19 show the results that confirm the sizes of exosomes after overexpressing exosomes in a cell line that is not transformed by a plasmid (FIG. 17), GNSTM-LAMP2-GFP (FIG. 18), and GNSTM-LAMP2-△EC/IC-GFP (FIG. 19).
FIG. 20 shows the results that confirm the expression levels of GNSTM-LAMP2-△EC/IC-shGFP (w/o BIV), GNSTM-LAMP2-ΔEC/IC-shGFP-BIV (w/BIV), GNSTM-LAMP2-△EC/IC-shGFP-JDV(WT) (JDV WT), and GNSTM-LAMP2-△EC/IC-shGFP-JDV(MT) (JDV_MT) in transfected cells and exosomes secreted therefrom.
FIG. 21 shows the qPCR results that confirm the expression levels of miRNA expressed in a cell line not transformed by a plasmid (Control), cell lines transfected by miRNA-free SEL-LAMP2-△EC/IC-RBP (w/o miRNA) and miRNA-containing SEL-LAMP2-△EC/IC-RBP (w/ miRNA), and target cells treated with exosomes secreted therefrom.
FIG. 22 shows the results that confirm GFP RNA expression inhibition levels upon the treatment of GFP-expressing cells with exosomes isolated from a cell line not transfected by a plasmid (Control), and cell lines transfected with GNSTM-LAMP2-△EC/IC-scrambleGFP (scramble GFP), GNSTM-LAMP2-△EC/IC-shGFP-BIV (BIV shGFP), GNSTM-LAMP2-△EC/IC-shGFP-JDV(WT) (JDV WT shGFP), and GNSTM-LAMP2-△EC/IC-shGFP-JDV(MT) (JDV MT shGFP), respectively.
FIG. 23 is the graph showing GFP expression inhibition levels quantified after Western blotting upon the treatment of GFP-expressing cells with exosomes isolated from a cell line not transfected by a plasmid (Control), and cell lines transfected with GNSTM-LAMP2-△EC/IC-scrambleGFP (scramble GFP), GNSTM-LAMP2-△EC/IC-shGFP-BIV (BIV shGFP), GNSTM-LAMP2-△EC/IC-shGFP-JDV(WT) (JDV WT shGFP), and GNSTM-LAMP2-△EC/IC-shGFP-JDV(MT) (JDV MT shGFP), respectively.
FIG. 24 is the Western blotting results of the graph of FIG. 23.
FIG. 25 shows the results that confirm GFP fluorescence expression inhibition levels, observed under a microscope, upon the treatment of GFP-expressing cells with exosomes isolated from a cell line not transfected by a plasmid (Control), and cell lines transfected with GNSTM-LAMP2-△EC/IC-scrambleGFP (scramble GFP), GNSTM-LAMP2-△EC/IC-shGFP-BIV (BIV shGFP), GNSTM-LAMP2-△EC/IC-shGFP-JDV(WT) (JDV WT shGFP), and GNSTM-LAMP2-△EC/IC-shGFP-JDV(MT) (JDV MT shGFP), respectively.

### [Modes of the Invention]

Hereinafter, the present invention will be described in detail with reference to examples and experimental examples. However, the following examples and experimental examples are merely illustrative, and the scope of the present invention is not limited thereto.

### Example 1. Production of recombinant plasmid containing gene encoding modified peptide

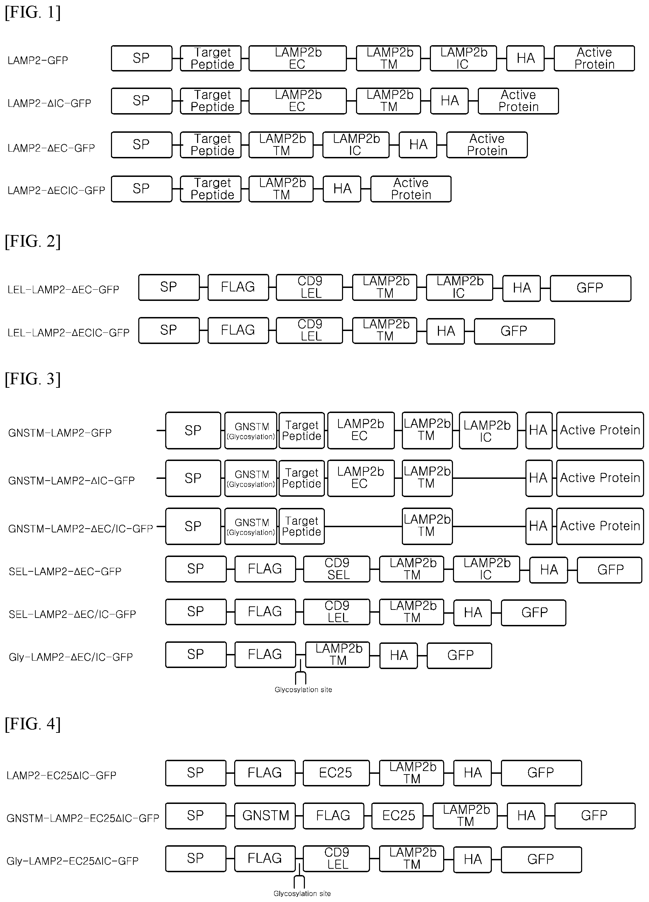

A LAMP2B-containing plasmid was produced by synthesizing the entire sequence encoding LAMP-2B (NM_013995.2), amplifying the sequence by PCR, and inserting the PCR product into a pcDNA 3.1 (+) vector (or can be inserted into an animal cell overexpression vector) in accordance with the method suggested in an infusion cloning kit (In-Fusion^{®} HD cloning kit, Clontech, Cat No. 639648). In accordance with the report presented by Michelle E. Hung et al. (The Journal of Biological Chemistry 2015. Mar 27), a recombinant plasmid was produced by synthesizing a hyaluronic acid (HA)-coding sequence, a target peptide-coding sequence, and an active protein-coding sequence, amplifying each sequence by PCR, and inserting the PCR products into the locations for the respective sequences using the infusion cloning kit (using SP: signal peptide, signal peptide-coding sequence; HA: hyaluronic acid, hyaluronic acid-coding sequence; Target Peptide: target peptide-coding sequence, and FLAG protein-coding sequence; Active Protein, GFP-coding sequence). In addition, like the method described above, a recombinant plasmid into which a sequence from which the intracellular domain (IC)-coding sequence of LAMP-2B was removed was inserted (LAMP2-△IC-GFP), a recombinant plasmid into which a sequence from which the extracellular domain (EC) of LAMP-2B was removed was inserted (LAMP2-△EC-GFP), and a recombinant plasmid into which a sequence from which both the intracellular domain and extracellular domain (EC) of LAMP-2B were removed was inserted (LAMP2-△EC/IC-GFP) were produced by the method suggested in the mutagenesis kit (EZchange Site-directed Mutagenesis kit, Enzynomics, Cat No. EZ004S). The results are schematically illustrated as shown in FIG. 1.

In addition, for some of the framework structures of FIG. 1, a CD9 large extracellular domain (CD9 LEL)-coding sequence in tetraspanin was synthesized and amplified by PCR, and then used to replace the extracellular domain (EC)-coding sequence of LAMP-2B using an infusion cloning kit, thereby producing recombinant plasmids (LEL-LAMP-△EC-GFP and LEL-LAMP-△EC/IC-GFP). These plasmids are illustrated in FIG. 2.

In addition, for some of the framework structures of FIG. 1, to introduce glycosylation in the upstream (extracellular direction) or downstream (intracellular direction) region of a targeting peptide, a recombinant plasmid was produced by ① introducing a sequence encoding a GNSTM motif (GNSTM is a sequence that is an amino acid sequence, which is most strongly N-glycosylated in cells) into a corresponding location using a mutagenesis kit (EZchange Site-directed Mutagenesis kit, Enzynomics, Cat No. EZ004S) (GNSTM-LAMP2-GFP, GNSTM-LAMP2-△IC-GFP, or GNSTM-LAMP2-△EC/IC-GFP), ② synthesizing a CD9 small extracellular domain (CD9 SEL)-coding sequence in tetraspanin, amplifying it by PCR, and introducing the resulting product into an existing LAMP2B-GFP modified plasmid using an infusion cloning kit (SEL-LAMP2-△EC-GFP or SEL-LAMP2-△EC/IC-GFP), or ③ introducing the N-glycosylated sequence AAC downstream of the sequence encoding the FLAG protein of LAMP2-βEC/IC-GFP using a mutagenesis kit (EZchange Site-directed Mutagenesis kit, Enzynomics, Cat No. EZ004S) (Gly-LAMP2-△EC/IC-GFP). These plasmids are illustrated in FIG. 3.

In addition, for some of the framework structures of FIG. 3, recombinant plasmids (LAMP2-EC25△IC-GFP, GNSTM-LAMP2-EC25△IC-GFP, and Gly-LAMP2-EC25ΔIC-GFP) into which a partial sequence (EC25) of the sequence encoding the extracellular domain of LAMP-2B was inserted using a mutagenesis kit (EZchange Site-directed Mutagenesis kit, Enzynomics, Cat No. EZ004S) were produced. These plasmids are illustrated in FIG. 4.

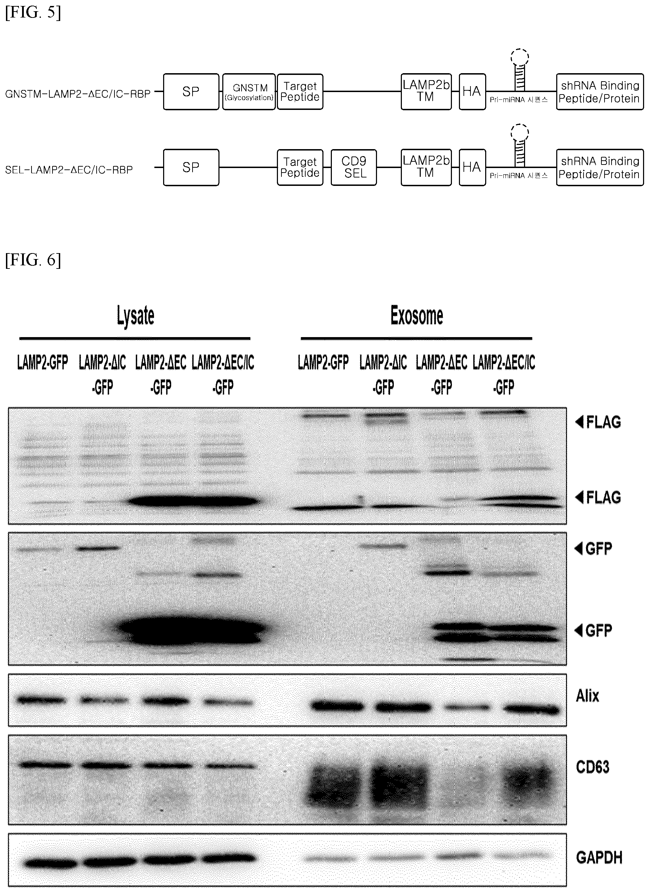

In addition, for some of the framework structures of FIG. 3, a sequence encoding an active protein in the GNSTM-LAMP-GFP-△EC/IC or SEL-LAMP2-ΔEC/IC was replaced with a sequence encoding a protein binding to shRNA, a pri-miRNA sequence was synthesized between an HA-coding sequence and the sequence encoding the shRNA-binding protein (RBP) and amplified by PCR, and then a recombinant plasmid (GNSTM-LAMP2-△EC/IC-RBP or SEL-LAMP2-△EC/IC-RBP) was produced using an infusion kit. These plasmids are illustrated in FIG. 5. As the pri-miRNA, the pri-miRNA sequence of shGFP in which a GFP target sequence is included in the pri-miRNA basic sequence of miRNA-199 or the miRNA-199 basic sequence was used. Since the TAR sequence is included in the pri-miRNA sequence structure, as an RBP, a protein specifically binding to the TAR site was intended to be introduced. Accordingly, as the proteins (RBPs: RNA binding proteins) binding to shRNA, a bovine immunodeficiency virus (BIV)-derived wild-type protein, and wild-type (WT) and mutant-type (MT) proteins of a Jembrana disease virus (JDV)-derived protein were used.

The specific sequences of the produced recombinant plasmids are shown in Table 1 below.

**[Table 1]**

| **Recombinant plasmid** | **Detailed sequence** |
|---|---|
| LAMP2 (SEQ ID NO: 1) | gagcatttcagataaatacctttgatctaagggttcagcctttcaatgtgacacaaggaaagtattctacagcccaagagtgttcgctggatgatg acaccattctaatcccaattatagttggtgctggtctttcaggcttgattatcgttatagtgattgcttacgtaattggcagaagaaaaagttatgctg gatatcagactctgTAA |
| LAMP2-ΔIC (SEQ ID NO: 2) | gagcatttcagataaatacctttgatctaagggttcagcctttcaatgtgacacaaggaaagtattctacagcccaagagtgttcgctggatgatg acaccattctaatcccaattatagttggtgctggtctttcaggcttgattatcgttatagtgattgcttacgtaattggcagaTAA |
| LAMP2-ΔEC (SEQ ID NO: 3) | atggtgtgcttccgcctcttcccggttccgggctcagggctcgttctggtctgcctagtcctgggagctgtgcggtcttatgcactaatcccaatt atagttggtgctggtctttcaggcttgattatcgttatagtgattgcttacgtaattggcagaagaaaaagttatgctggatatcagactctgTAA |
| LAMP2-ΔEC/IC (SEQ ID NO: 4) | atggtgtgcttccgcctcttcccggttccgggctcagggctcgttctggtctgcctagtcctgggagctgtgcggtcttatgcactaatcccaatt atagttggtgctggtctttcaggcttgattatcgttatagtgattgcttacgtaattggcagaTAA |
| LAMP2-GFP (SEQ ID NO: 5) | GGACGAGCTGTACAAGTAA |
| LAMP2-ΔIC-GFP (SEQ ID NO: 6) | tgtgcaacaaagagcagactgtttcagtgtctggagcatttcagataaatacctttgatctaagggttcagcctttcaatgtgacacaaggaaagt attctacagcccaagagtgttcgctggatgatgacaccattctaatcccaattatagttggtgctggtctttcaggcttgattatcgttatagtgattg cttacgtaattggcagaGGTAGCGGTggaagcggatacccatacgatgtgccagattacgctAAGTCGACGGTACCG CGGGCCCGGGATCCACCGGTCGCCACCATGGTGAGCAAGGGCGAGGAGCTGTTCACC GGGGTGGTGCCCATCCTGGTCGAGCTGGACGGCGACGTAAACGGCCACAAGTTCAGC GTGTCCGGCGAGGGCGAGGGCGATGCCACCTACGGCAAGCTGACCCTGAAGTTCATC TGCACCACCGGCAAGCTGCCCGTGCCCTGGCCCACCCTCGTGACCACCCTGACCTAC GGCGTGCAGTGCTTCAGCCGCTACCCCGACCACATGAAGCAGCACGACTTCTTCAAG TCCGCCATGCCCGAAGGCTACGTCCAGGAGCGCACCATCTTCTTCAAGGACGACGGC AACTACAAGACCCGCGCCGAGGTGAAGTTCGAGGGCGACACCCTGGTGAACCGCATC GAGCTGAAGGGCATCGACTTCAAGGAGGACGGCAACATCCTGGGGCACAAGCTGGA GTACAACTACAACAGCCACAACGTCTATATCATGGCCGACAAGCAGAAGAACGGCAT |
| | CAAGGTGAACTTCAAGATCCGCCACAACATCGAGGACGGCAGCGTGCAGCTCGCCGA CCACTACCAGCAGAACACCCCCATCGGCGACGGCCCCGTGCTGCTGCCCGACAACCA CTACCTGAGCACCCAGTCCGCCCTGAGCAAAGACCCCAACGAGAAGCGCGATCACAT GGTCCTGCTGGAGTTCGTGACCGCCGCCGGGATCACTCTCGGCATGGACGAGCTGTA CAAGTAA |
| LAMP2-ΔEC-GFP (SEQ ID NO: 7) | AGCTGTACAAGTAA |
| LAMP2-ΔEC/IC-GFP (SEQ ID NO: 8) | atggtgtgcttccgcctcttcccggttccgggctcagggctcgttctggtctgcctagtcctgggagctgtgcggtcttatgcagactataaagat gacgatgacaagggctcgagtGgtggaggatctggtctaatcccaattatagttggtgctggtctttcaggcttgattatcgttatagtgattgctt acgtaattggcagaGGTAGCGGTggaagcggatacccatacgatgtgccagattacgctAAGTCGACGGTACCGCG GGCCCGGGATCCACCGGTCGCCACCATGGTGAGCAAGGGCGAGGAGCTGTTCACCGG GGTGGTGCCCATCCTGGTCGAGCTGGACGGCGACGTAAACGGCCACAAGTTCAGCGT GTCCGGCGAGGGCGAGGGCGATGCCACCTACGGCAAGCTGACCCTGAAGTTCATCTG CACCACCGGCAAGCTGCCCGTGCCCTGGCCCACCCTCGTGACCACCCTGACCTACGG CGTGCAGTGCTTCAGCCGCTACCCCGACCACATGAAGCAGCACGACTTCTTCAAGTC CGCCATGCCCGAAGGCTACGTCCAGGAGCGCACCATCTTCTTCAAGGACGACGGCAA CTACAAGACCCGCGCCGAGGTGAAGTTCGAGGGCGACACCCTGGTGAACCGCATCGA GCTGAAGGGCATCGACTTCAAGGAGGACGGCAACATCCTGGGGCACAAGCTGGAGT ACAACTACAACAGCCACAACGTCTATATCATGGCCGACAAGCAGAAGAACGGCATC AAGGTGAACTTCAAGATCCGCCACAACATCGAGGACGGCAGCGTGCAGCTCGCCGAC CACTACCAGCAGAACACCCCCATCGGCGACGGCCCCGTGCTGCTGCCCGACAACCAC TACCTGAGCACCCAGTCCGCCCTGAGCAAAGACCCCAACGAGAAGCGCGATCACATG GTCCTGCTGGAGTTCGTGACCGCCGCCGGGATCACTCTCGGCATGGACGAGCTGTAC AAGTAA |
| LEL-LANIP2-ΔEC-GFP (SEQ ID NO: 9) | atggtgtgcttccgcctcttcccggttccgggctcagggctcgttctggtctgcctagtcctgggagctgtgcggtcttatgcagactataaagat gacgatgacaagggctcgagtGgtggaggatctggtTATTCCCACAAGGATGAGGTGATTAAGGAAGTCC AGGAGTTTTACAAGGACACCTACAACAAGCTGAAAACCAAGGATGAGCCCCAGCGG GAAACGCTGAAAGCCATCCACTATGCGTTGAACTGCTGTGGTTTGGCTGGGGGCGTG GAACAGTTTATCTCAGACATCTGCCCCAAGAAGGACGTACTCGAAACCTTCACCGTG AAGTCCTGTCCTGATGCCATCAAAGAGGTCTTCGACctaatcccaattatagttggtgctggtctttcaggctt gattatcgttatagtgattgcttacgtaattggcagaagaaaaagttatgctggatatcagactctgggaagcggatacccatacgatgtgccag attacgcttaa |
| LEL-LAMP2-ΔEC/IC-GFP (SEQ ID NO: 10) | atggtgtgcttccgcctcttcccggttccgggctcagggctcgttctggtctgcctagtcctgggagctgtgcggtcttatgcagactataaagat gacgatgacaagggctcgagtGgtggaggatctggtTATTCCCACAAGGATGAGGTGATTAAGGAAGTCC AGGAGTTTTACAAGGACACCTACAACAAGCTGAAAACCAAGGATGAGCCCCAGCGG GAAACGCTGAAAGCCATCCACTATGCGTTGAACTGCTGTGGTTTGGCTGGGGGCGTG GAACAGTTTATCTCAGACATCTGCCCCAAGAAGGACGTACTCGAAACCTTCACCGTG AAGTCCTGTCCTGATGCCATCAAAGAGGTCTTCGACctaatcccaattatagttggtgctggtctttcaggctt gattatcgttatagtgattgcttacgtaattggcagaGGTAGCGGTggaagcggatacccatacgatgtgccagattacgcttaa |
| GNSTM (SEQ ID NO: 11) | GGTAACTCGACTATG |
| SEL (SEQ ID NO: 12) | CTCCGATTCGACTCTCAGACCAAGAGCATCTTCGAGCAAGAAACTAATAATAATAAT TCCAGCTTCTAC |
| Gly (SEQ ID NO: 13) | AAC |
| GNSTM-LAMP2-GFP (SEQ ID NO: 14) | GGTCCTGCTGGAGTTCGTGACCGCCGCCGGGATCACTCTCGGCATGGACGAGCTGTA CAAGTAA |
| GNSTM-LAMP2-ΔIC-GFP (SEQ ID NO: 15) | CGCCGCCGGGATCACTCTCGGCATGGACGAGCTGTACAAGTAA |
| GNSTM-LAMP2-ΔEC/IC-GFP (SEQ ID NO: 16) | GCATGGACGAGCTGTACAAGTAA |
| SEL-LAMP2-ΔEC-GFP (SEQ ID NO: 17) | AAGACCCCAACGAGAAGCGCGATCACATGGTCCTGCTGGAGTTCGTGACCGCCGCCG GGATCACTCTCGGCATGGACGAGCTGTACAAGtaa |
| SEL-LAMP2-ΔEC/IC-GFP (SEQ ID NO: 18) | ATCACATGGTCCTGCTGGAGTTCGTGACCGCCGCCGGGATCACTCTCGGCATGGACG AGCTGTACAAGtaa |
| Gly-LAMP2-ΔEC/IC-GFP (SEQ ID NO: 19) | atggtgtgcttccgcctcttcccggttccgggctcagggctcgttctggtctgcctagtcctgggagctgtgcggtcttatgcagactataaagat gacgatgacaagggctcgagtGgtAACggatctggtctaatcccaattatagttggtgctggtctttcaggcttgattatcgttatagtgattg cttacgtaattggcagaGGTAGCGGTggaagcggatacccatacgatgtgccagattacgctAAGTCGACGGTACCG CGGGCCCGGGATCCACCGGTCGCCACCATGGTGAGCAAGGGCGAGGAGCTGTTCACC GGGGTGGTGCCCATCCTGGTCGAGCTGGACGGCGACGTAAACGGCCACAAGTTCAGC GTGTCCGGCGAGGGCGAGGGCGATGCCACCTACGGCAAGCTGACCCTGAAGTTCATC TGCACCACCGGCAAGCTGCCCGTGCCCTGGCCCACCCTCGTGACCACCCTGACCTAC GGCGTGCAGTGCTTCAGCCGCTACCCCGACCACATGAAGCAGCACGACTTCTTCAAG TCCGCCATGCCCGAAGGCTACGTCCAGGAGCGCACCATCTTCTTCAAGGACGACGGC AACTACAAGACCCGCGCCGAGGTGAAGTTCGAGGGCGACACCCTGGTGAACCGCATC GAGCTGAAGGGCATCGACTTCAAGGAGGACGGCAACATCCTGGGGCACAAGCTGGA GTACAACTACAACAGCCACAACGTCTATATCATGGCCGACAAGCAGAAGAACGGCAT CAAGGTGAACTTCAAGATCCGCCACAACATCGAGGACGGCAGCGTGCAGCTCGCCGA CCACTACCAGCAGAACACCCCCATCGGCGACGGCCCCGTGCTGCTGCCCGACAACCA CTACCTGAGCACCCAGTCCGCCCTGAGCAAAGACCCCAACGAGAAGCGCGATCACAT GGTCCTGCTGGAGTTCGTGACCGCCGCCGGGATCACTCTCGGCATGGACGAGCTGTA CAAGTAA |
| LAMP2-EC25 ΔIC-GFP (SEQ ID NO: 20) | ACATGGTCCTGCTGGAGTTCGTGACCGCCGCCGGGATCACTCTCGGCATGGACGAGC TGTACAAGtaa |
| GNSTM-LAMP2-EC25 ΔIC-GFP (SEQ ID NO: 21) | TGAGCAAAGACCCCAACGAGAAGCGCGATCACATGGTCCTGCTGGAGTTCGTGACCG CCGCCGGGATCACTCTCGGCATGGACGAGCTGTACAAGTAA |
| Gly-LAMP2-EC25 ΔIC-GFP (SEQ ID NO: 22) | ACATGGTCCTGCTGGAGTTCGTGACCGCCGCCGGGATCACTCTCGGCATGGACGAGC TGTACAAGtaa |
| GNSTM-LAMP2-ΔEC/IC-shGFP (SEQ ID NO: 23) | ATGGTGTGCTTCCGCCTCTTCCCGGTTCCGGGCTCAGGGCTCGTTCTGGTCTGCCTAG TCCTGGGAGCTGTGCGGTCTTATGCAGGTAACTCGACTATGGGCAGTGGAGACTATA AAGATGACGATGACAAGGGCAGTGGATCTGGATCCGGTGGCTCGAGTCTAATCCCAA TTATAGTTGGTGCTGGTCTTTCAGGCTTGATTATCGTTATAGTGATTGCTTACGTAATT GGCAGAGGTAGCGGTggaagcggatacccatacgatgtgccagattacgctGGATCGGGTAGGCACCAGgt aggggagctggctgggtggggcagccccgggaaggggggaaagctgaatgcaacccctggtgcaggaagggaggcttttcctgaggac cgggagaggattttaagtacatagaaggaagcttctggagatcctgctccgtcgcCGTCTATATCATGGCCGACATCTC TGAGCCTGGGAGCTCTGTCGGCCATGATATAGACGTTAgactgggcaagggagagcaacgccatgga ccgctggggacaaaatgggctgtttccaaggagaagacatttgtttgctccttttttgattcttgtcctttccttcccagGGCGTGATGGG CAGCGGCTAA |
| GNSTM-LAMP2-ΔEC/IC-shGFP-BIV (SEQ ID NO: 24) | ATGGTGTGCTTCCGCCTCTTCCCGGTTCCGGGCTCAGGGCTCGTTCTGGTCTGCCTAG TCCTGGGAGCTGTGCGGTCTTATGCAGGTAACTCGACTATGGGCAGTGGAGACTATA AAGATGACGATGACAAGGGCAGTGGATCTGGATCCGGTGGCTCGAGTCTAATCCCAA TTATAGTTGGTGCTGGTCTTTCAGGCTTGATTATCGTTATAGTGATTGCTTACGTAATT GGCAGAGGTAGCGGTggaagcggatacccatacgatgtgccagattacgctGGATCGGGTAGGCACCAG g tag gggagctggctgggtggggcagccccgggaaggggggaaagctgaatgcaacccctggtgcaggaagggaggcttttcctgagg accgggagaggattttaagtacatagaaggaagcttctggagatcctgctccgtcgcCGTCTATATCATGGCCGACATC TCTGAGCCTGGGAGCTCTGTCGGCCATGATATAGACGTTAgactgggcaagggagagcaacgccat ggaccgctggggacaaaatgggctgtttccaaggagaagacatttgtttgctccttttttgattcttgtcctttccttcccagGGCGTGATG GGCAGCGGCGGCAGCGGCCGACCACGTGGGACACGCGGCAAGGGCCGGCGCATTAG GCGGTAA |
| GNSTM-LAMP2-ΔEC/IC-shGFP-JDV(WT) (SEQ ID NO: 25) | ATGGTGTGCTTCCGCCTCTTCCCGGTTCCGGGCTCAGGGCTCGTTCTGGTCTGCCTAG TCCTGGGAGCTGTGCGGTCTTATGCAGGTAACTCGACTATGGGCAGTGGAGACTATA AAGATGACGATGACAAGGGCAGTGGATCTGGATCCGGTGGCTCGAGTCTAATCCCAA TTATAGTTGGTGCTGGTCTTTCAGGCTTGATTATCGTTATAGTGATTGCTTACGTAATT GGCAGAGGTAGCGGTggaagcggatacccatacgatgtgccagattacgctGGATCGGGTAGGCACCAGgt aggggagctggctgggtggggcagccccgggaaggggggaaagctgaatgcaacccctggtgcaggaagggaggcttttcctgaggac cgggagaggattttaagtacatagaaggaagcttctggagatcctgctccgtcgcCGTCTATATCATGGCCGACATCTC TGAGCCTGGGAGCTCTGTCGGCCATGATATAGACGTTAgactgggcaagggagagcaacgccatgga |
| | ccgctggggacaaaatgggctgtttccaaggagaagacatttgtttgctccttttttgattcttgtcctttccttcccagGGCGTGATGGG CAGCGGCGGCAGCGGCCGACGAAAAAAGCGTGGGACACGCGGCAAGGGCCGGAAG ATTCACTATTAACGGTAA |
| GNSTM-LAMP2-ΔEC/IC-shGFP-JDV(MT) (SEQ ID NO: 26) | ATGGTGTGCTTCCGCCTCTTCCCGGTTCCGGGCTCAGGGCTCGTTCTGGTCTGCCTAG TCCTGGGAGCTGTGCGGTCTTATGCAGGTAACTCGACTATGGGCAGTGGAGACTATA AAGATGACGATGACAAGGGCAGTGGATCTGGATCCGGTGGCTCGAGTCTAATCCCAA TTATAGTTGGTGCTGGTCTTTCAGGCTTGATTATCGTTATAGTGATTGCTTACGTAATT GGCAGAGGTAGCGGTggaagcggatacccatacgatgtgccagattacgctGGATCGGGTAGGCACCAGgt aggggagctggctgggtggggcagccccgggaaggggggaaagctgaatgcaacccctggtgcaggaagggaggcttttcctgaggac cgggagaggattttaagtacatagaaggaagcttctggagatcctgctccgtcgcCGTCTATATCATGGCCGACATCTC TGAGCCTGGGAGCTCTGTCGGCCATGATATAGACGTTAgactgggcaagggagagcaacgccatgga ccgctggggacaaaatgggctgtttccaaggagaagacatttgtttgctccttttttgattcttgtcctttccttcccagGGCGTGATGGG CAGCGGCGGCAGCGGCCGACGAAAAAAGCGTGGGACACGCGGCAAGGGCCGGCGCA TTAGGCGGTAA |
| SEL-LAMP2-ΔEC/IC-BIV (SEQ ID NO: 27) | atggtgtgcttccgcctcttcccggttccgggctcagggctcgttctggtctgcctagtcctgggagctgtgcggtcttatgcagactataaagat gacgatgacaagggctcgagtGgtggaggatctggtCTCCGATTCGACTCTCAGACCAAGAGCATCTTCG AGCAAGAAACTAATAATAATAATTCCAGCTTCTACACAGGAGTCctaatcccaattatagttggtgc tggtctttcaggcttgattatcgttatagtgattgcttacgtaattggcagaGGTAGCGGTggaagcggatacccatacgatgtgccaga ttacgctGGCAGCGGCGGCAGCGGCCGACCACGTGGGACACGCGGCAAGGGCCGGCGCA TTAGGCGGtaa |
| SEL-LAMP2-ΔEC/IC-(pri-miRNA-199)-BIV (SEQ ID NO: 28) | atggtgtgcttccgcctcttcccggttccgggctcagggctcgttctggtctgcctagtcctgggagctgtgcggtcttatgcagactataaagat gacgatgacaagggctcgagtGgtggaggatctggtCTCCGATTCGACTCTCAGACCAAGAGCATCTTCG AGCAAGAAACTAATAATAATAATTCCAGCTTCTACACAGGAGTCctaatcccaattatagttggtgc tggtctttcaggcttgattatcgttatagtgattgcttacgtaattggcagaGGTAGCGGTggaagcggatacccatacgatgtgccaga ttacgctGGATCGGGTAGGCACCAGgtaggggagctggctgggtggggcagccccgggaaggggggaaagctgaatgc aacccctggtgcaggaagggaggcttttcctgaggaccgggagaggattttaagtacatagaaggaagcttctggagatcctgctccgtcgc CCCAGTGTTCAGACTACCTGTTCTCTGAGCCTGGGAGCTCACAGTAGTCTGCACATTG GTTAgactgggcaagggagagcaacgccatggaccgctggggacaaaatgggctgtttccaaggagaagacatttgtttgctccttttttg attcttgtcctttccttcccagGGCGTGATGGGCAGCGGCGGCAGCGGCCGACCACGTGGGACACG CGGCAAGGGCCGGCGCATTAGGCGGTAA |
| GNSTM-LAMP2-ΔEC/IC-scramble shGFP (SEQ ID NO: 29) | ATGGTGTGCTTCCGCCTCTTCCCGGTTCCGGGCTCAGGGCTCGTTCTGGTCTGCCTAG TCCTGGGAGCTGTGCGGTCTTATGCAGGTAACTCGACTATGGGCAGTGGAGACTATA AAGATGACGATGACAAGGGCAGTGGATCTGGATCCGGTGGCTCGAGTCTAATCCCAA TTATAGTTGGTGCTGGTCTTTCAGGCTTGATTATCGTTATAGTGATTGCTTACGTAATT GGCAGAGGTAGCGGTGGAAGCGGATACCCATACGATGTGCCAGATTACGCTGGATCG GGTAGGCACCAGgtaggggagctggctgggtggggcagccccgggaaggggggaaagctgaatgcaacccctggtgcagg aagggaggcttttcctgaggaccgggagaggattttaagtacatagaaggaagcttctggagatcctgctccgtcgcCTAAGGTTAA GTCGCCCTCGTCTCTGAGCCTGGGAGCTCCGAGGGCGACTTAACCTTAGTTAgactgggca agggagagcaacgccatggaccgctggggacaaaatgggctgtttccaaggagaagacatttgtttgctccttttttgattcttgtcctttccttc ccagGGCGTGATGGGCAGCGGCTAA |
| Double stranded RNA Binding Domain motif (DRBD) (SEQ ID NO: 30) | TTCTTCATGGAGGAACTTAATACATACCGTCAGAAGCAGGGAGTAGTACTTAAATAT CAAGAACTGCCTAATT CAGGACCTCCACATGATAGGAGGTTTACATTTCAAGTTATAATAGATGGAAGAGAAT TTCCAGAAGGTGA AGGTAGATCAAAGAAGGAAGCAAAAAATGCCGCAGCCAAATTAGCTGTTGAGATAC TTAATAAGGAA |
| BIV-derived RBD(BIV) (SEQ ID NO: 31) | CGACCACGTGGGACACGCGGCAAGGGCCGGCGCATTAGGCGG |
| JDV-derived RBD(JDV(WT)) (SEQ ID NO: 32) | CGACGAAAAAAGCGTGGGACACGCGGCAAGGGCCGGAAGATTCACTATTAA |
| JDV-derived variant RBD(JDV(MT)) (SEQ ID NO: 33) | CGACGAAAAAAGCGTGGGACACGCGGCAAGGGCCGGCGCATTAGGCGG |
| HIV-derived RBD (SEQ ID NO: 34) | AGGAAGAAGAGGAGGCAGAGGAGGAGG |

### Example 2. Expression of recombinant plasmids

To express a recombinant plasmid, 2.5×10⁶ HEK293T cells were cultured in a 100 mm² culture plate containing DMEM (Welgene, Cat No. LM001-05) supplemented with 10% fetal bovine serum (FBS, Gibco^{™}, Cat No. 16000044) and 1% antibiotics (1% penicillin/streptomycin, Gibco^{™}, Cat No. 15140122) for 24 hours. 2 µg of a recombinant plasmid containing a target gene, such as the pcDNA3.1(+) vector, was introduced into the cells according to the method of a PolyJet^{™}transfection kit (SignaGen^{®} Laboratories, Cat No. SL100688), and incubated for 48 hours.

### Example 2-1. Fluorescence imaging experiment

To confirm the intracellular fluorescence expression of the recombinant plasmid, the medium was exchanged with FBS-free DMEM and then the cells were further incubated for 48 hours. After the incubation was completed, green fluorescence expression in cells was confirmed using Cytation 5 (Biotek).

### Example 2-2. Exosome isolation, protein expression, and RNA expression

After washing the cells once with 1XDPBS (Welgene, Cat No. LB001-02), 1 mL of trypsin-EDTA (Welgene, Cat No. LS015-10) was added to the culture plate, the cells were detached from the culture plate, 10 mL of DMEM containing FBS and antibiotics was added to collect the cells, followed by centrifuging at 1000 rpm for 2 minutes. The cells collected by removing the medium were resuspended in 10 mL of FBS-free DMEM, and then added to a suspension culture dish (SPL Life Sciences, Cat No. 11151) to further culture in 1% antibiotics-containing DMEM for 48 hours. Subsequently, exosomes and the cells were isolated to perform experiments for protein expression and RNA expression.

### Experimental Example 1. Stable expression and delivery of active protein to target using modified peptide

### 1. Confirmation of effect of increasing target protein expression level after removing intracellular domain (IC) and extracellular domain (EC)

### (1) Experimental method

After inserting a FLAG protein-coding gene as a targeting peptide and a GFP-coding gene as an active protein into the plasmid framework structures of FIGS. 1 and 2, respectively, the recombinant plasmids were overexpressed by the method described in Example 2-1 or 2-2.

That is, to confirm the actual fluorescence expression of cells, each recombinant plasmid was overexpressed by the method of Example 2-1, and then fluorescence expression was measured.

In addition, to confirm a protein expression level, after completing the culture according to Example 2-2, the cells and the medium were collected in a 50 mL conical tube (SPL, Cat No. 50040), centrifuged at 1000 rpm for 2 minutes, the collected cells were washed twice with 1X PBS, and PMSF was added to 10X cell lysis buffer (Cell Signaling Technology, Cat No. 9803) diluted to 1X to isolate the protein from the cells. Afterward, the isolated culture solution was additionally centrifuged at 4000 rpm for 30 minutes, cell debris was removed, and the supernatant was added to a 10kDa filter tube (Millipore, Cat No. UFC9010) and centrifuged at 4000 rpm for 30 minutes. Finally, 1X PBS was added to the remaining supernatant and mixed and then further centrifuged. After repeating this process twice, the supernatant remaining in the filter tube was filtered through a 0.2 µm filter using a syringe with a 27-G needle, thereby obtaining exosomes of a single size. The cell lysate and the obtained exosomes were subjected to Western blotting to compare the expression levels of recombinant proteins in the cells and the exosomes.

### (2) Experimental results

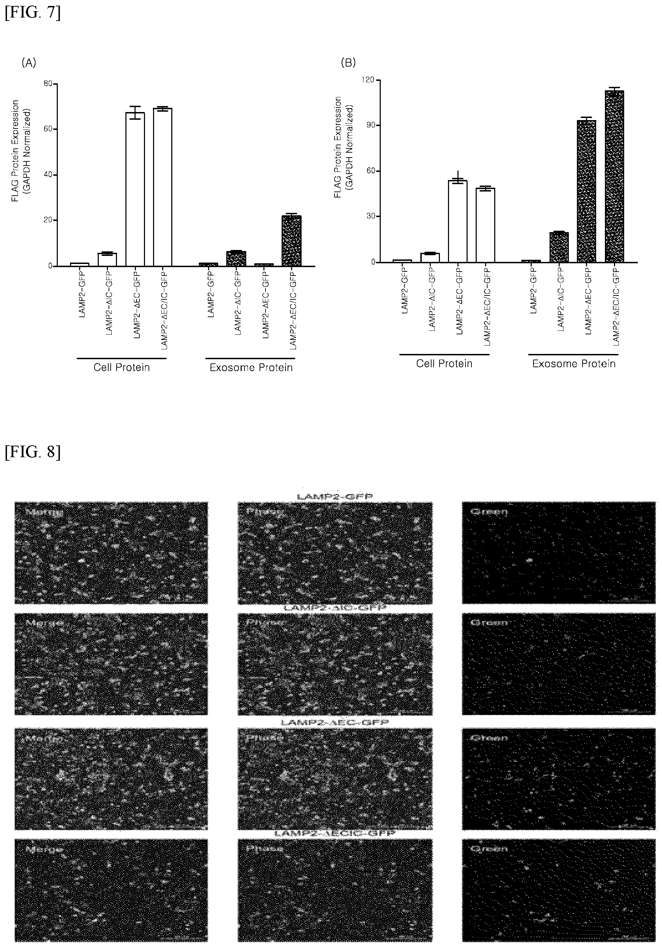

In the case of overexpression of the plasmids of FIG. 1, compared to when the recombinant plasmid into which the entire sequence encoding LAMP-2B was inserted (LAMP2-GFP) was overexpressed, when the recombinant plasmid from which a sequence encoding the intracellular domain (LAMP2-△IC-GFP), the extracellular domain (LAMP2-△EC-GFP), or both (LAMP2-△EC/IC-GFP) were removed was overexpressed, the expression levels of the FLAG protein and GFP were significantly high (FIGS. 6 and 7). Particularly, in LAMP2-△EC/IC-GFP, compared to LAMP2-GFP, the expression level of the FLAG protein increased approximately 6 to 20 times and the expression level of the GFP protein increased approximately 50 to 100 times in cells and exosomes (FIGS. 6A and 6B). The actual fluorescence measurement results can also show that the intensity of fluorescence was significantly stronger in LAMP2-△IC-GFP, LAMP2-△EC-GFP, and LAMP2-△EC/IC-GFP compared to LAMP2-GFP, and particularly, it can be confirmed that the fluorescence intensity in LAMP2-△EC/IC-GFP was the strongest (FIG. 8).

This demonstrates that removing the intracellular domain of LAMP2 can increase the stable expression of both the target protein and the active protein in cells that secrete exosomes or the exosomes, and furthermore, this shows that the stability of expression can be significantly increased by removing the extracellular domain of LAMP2. LAMP2 is known to be a protein involved in lysosomal degradation, and it is assumed that the lysosomal degradation is avoided by removing the intracellular domain of LAMP2 to increase the expression rate of target proteins. However, when the extracellular domain of LAMP2 was also removed, as the stability of LAMP2 itself was significantly lowered, it was expected to play a negative role in the expression rates of target proteins, but a significant increase in expression rate was an unexpected effect. It seems that there is a need for further research on this.

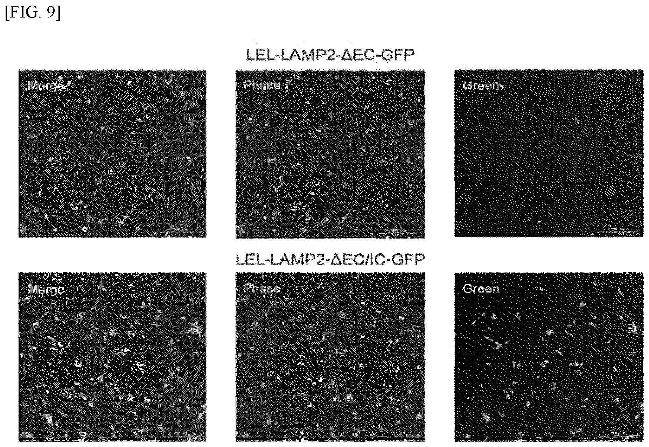

In addition, in the case of the overexpression of the plasmids of FIG. 2, compared to when the sequence encoding the intracellular domain of LAMP2 was not removed (LEL-LAMP2-△EC), when the sequence was removed (LEL-LAMP2-△EC/IC), it can be confirmed that the fluorescence intensity was significantly stronger (FIG. 9).

This shows more clearly that, when the intracellular domain of LAMP2 was removed, the expression level of a target protein may be increased by inhibiting the lysosomal degradation of cells due to the intracellular influx of foreign proteins by the intracellular domain. Even when the extracellular domain ofLAMP2 was replaced with the extracellular domain of another protein, the same result was shown.

### 2. Confirmation of effect of increasing expression level by glycosylation

### (1) Experimental method

A FLAG protein-coding gene as a targeting peptide, and a GFP-coding gene as an active protein were inserted into the plasmid framework structures of FIGS. 3 and 4, respectively, and the recombinant plasmids were overexpressed by the methods of Examples 2-1 and 2-2, respectively. Other than this, the experimental method was the same as described in Experimental Example 1-1-(1) above.

### (2) Experimental results

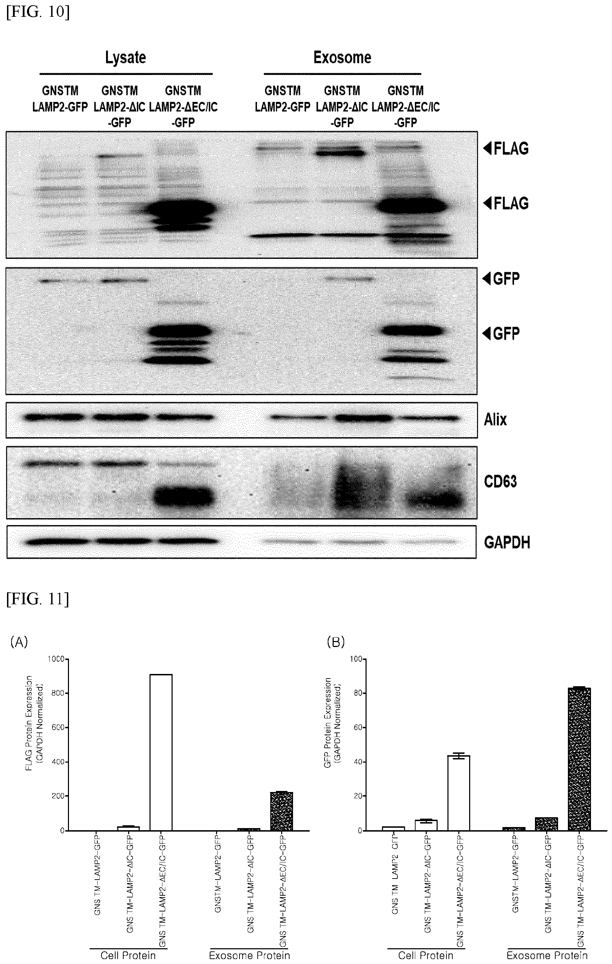

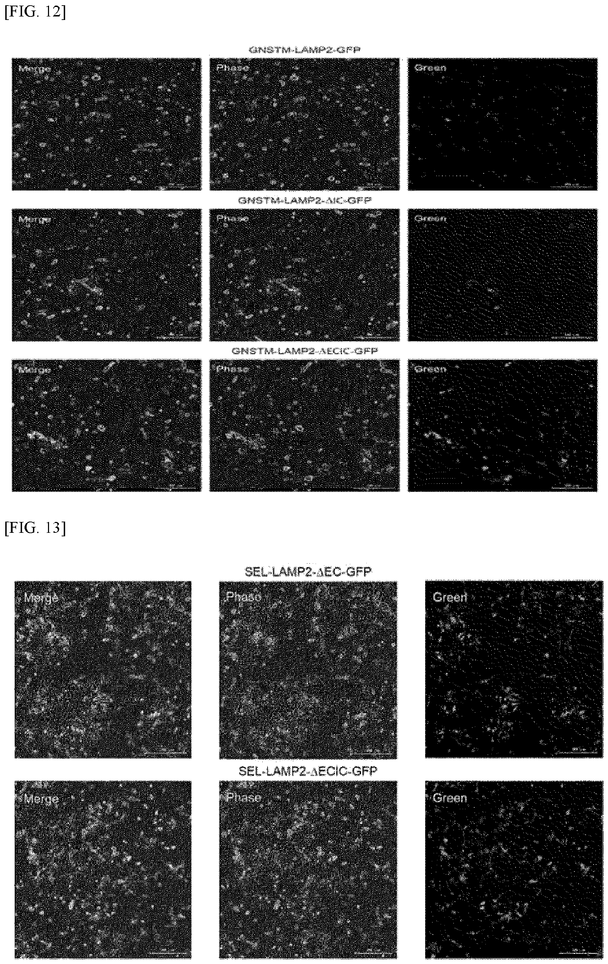

When the plasmids of FIG. 3 were overexpressed, in the plasmid in which the entire sequence of LAMP2 is included in a GNSTM motif sequence (GNSTM-LAMP2-GFP), the expression levels of FLAG protein as a target protein and GFP as an active protein were very low (FIG. 10). This is a result inconsistent with that reported in existing papers, meaning that the GNSTM motif does not substantially provide an effect of stabilizing a target protein in LAMP2. However, when the intracellular domain of LAMP2 was removed (GNSTM-LAMP2-△IC-GFP), or the extracellular domain thereof was also removed (GNSTM-LAMP2-△EC/IC-GFP), it resulted in the GNSTM motif greatly stabilizing the expression of target proteins. Particularly, when both the intracellular domain and extracellular domain of LAMP2 were removed (GNSTM-LAMP2-△EC/IC-GFP), the expression levels of both the FLAG protein as a target protein and GFP as an active protein were significantly high. Compared to when the GNSTM motif sequence bound to the entire sequence of LAMP2, the expression level of FLAG increased 200 to 900 times, and the expression level of GFP increased 40 to 80 times (FIG. 11). As the fluorescence levels also reflect the above results, the fluorescence intensity in GNSTM-LAMP2-△IC-GFP was stronger than that of GNSTM-LAMP2-GFP, and GNSTM-LAMP2-△EC/IC-GFP more clearly showed a significant difference in fluorescence intensity (FIG. 12).

This tendency was the same even when the CD9 SEL domain, conventionally known to contain a glycosylated amino acid, was included downstream of the target protein. Compared to when the extracellular domain of LAMP2 was replaced with SEL (SEL-LAMP2-△EC-GFP), when the intracellular domain thereof was also removed (SEL-LAMP2-△EC/IC-GFP), the fluorescence intensity was more strongly shown (FIG. 13).

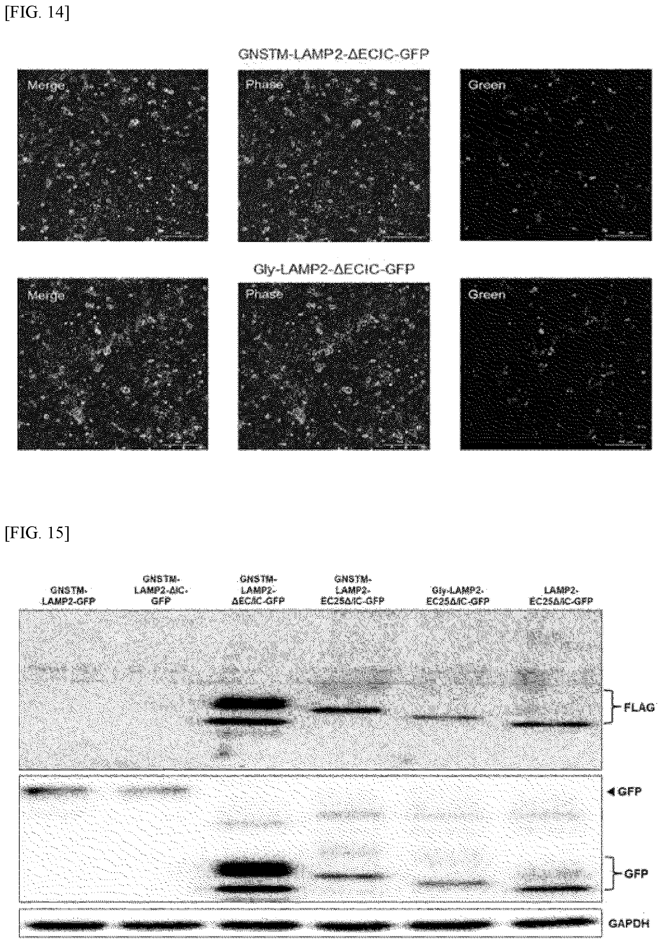

In addition, in the LAMP2-△EC/IC-GFP plasmid, a DNA sequence was substituted at the corresponding location using a mutagenesis kit to substitute the glycosylated active amino acid "glycine-asparagine-glycine (GNG)" in the intermediate region of a linker located downstream of FLAG. That is, an amino acid sequence "GSSGGGSG (DNA sequence: ggctcgagtGgtggaggatctggt)" of the linker was modified into "GSSGNGSG (DNA sequence: ggctcgagtGgtAACggatctggt)." As a result, even when glycosylation was performed downstream of the target protein (Gly-LAMP2-△EC/IC-GFP), the same tendency was shown, and when both the extracellular domain and intracellular domain of LAMP2 were removed, like when the GNSTM motif and SEL were introduced, it can be confirmed that the fluorescence intensity was very strong (FIG. 14).

In addition, with the expectation that including a part of the extracellular domain of LAMP2 could further increase the stabilization of the target protein depending on the location and structure of glycosylation, plasmids into which a nucleic acid sequence encoding a downstream 25-amino acid sequence of LAMP2 (LAMP2-EC25△IC-GFP, GNSTM-LAMP2-EC25△IC-GFP, and Gly-LAMP2-EC25△IC-GFP) were further produced, and then the expression level of the target protein was confirmed. As a result, compared to GNSTM-LAMP2-GFP, in all of GNSTM-LAMP2-EC25△IC-GFP, Gly-LAMP2-EC25△IC-GFP, and GNSTM-LAMP2-△EC/IC-GFP, the expression levels of the FLAG protein as the target protein and GFP as an active protein were high, and particularly, they were significantly high in GNSTM-LAMP2-△EC/IC-GFP. That is, this shows that, under the same tendency, when both the extracellular domain and intracellular domain of LAMP2 were removed, the highest expression effect of the target protein was shown. In addition, in the case of LAMP2-EC25△IC-GFP, a higher expression level of the target protein was shown. It is considered that this shows that the GNSTM motif does not exhibit an expression stabilization effect in the presence of the extracellular domain of LAMP2 (FIG. 15).

This shows that, in order for the conventionally known GNSTM motif to work together with LAMP2 to exhibit the expression stabilization effect of the target protein, at least the intracellular domain of LAMP2 should be removed from the motif, and when the extracellular domain thereof was also removed, the expression level of a target protein was significantly increased, which is a result that cannot be easily predicted from what was conventionally known.

### 3. Cancer cell targeting using exosomes

### (1) Experimental method

Among the plasmid framework structures of FIG. 3, in the GNSTM-containing plasmid, it was confirmed that FLAG expression levels of GNSTM-LAMP2-△EC/IC-GFP were significantly increased in cells and exosomes. After substituting a nucleic acid sequence encoding a cancer targeting peptide sequence ("MHTAPGWGYNLS"; folate receptor binding peptide) at the target peptide location of the plasmid (Table 2 below), each recombinant plasmid was overexpressed according to the method shown in Example 2-2, and after completing the culture, the cells and the culture solution were collected in a 50 mL conical tube (SPL, Cat No. 50040) and centrifuged at 1000 rpm for 2 minutes. After adding the supernatant, cell debris was removed by centrifugation at 4000 rpm for 30 minutes. The final supernatant was added to a 10kDa filter tube (Millipore, Cat No. UFC9010), and centrifuged at 4000 rpm for 30 minutes. Finally, the remaining supernatant was mixed with 1X PBS and further centrifuged, this process was repeated twice, and the supernatant remaining in the filter tube was filtered through a 0.2 µm filter using a syringe with a 27-G needle, thereby obtaining exosomes of a single size. The obtained exosomes were quantified using a BCA kit, and 100 µg of the exosomes were stained according to a method of a PKH67 green fluorescence cell linker kit (Sigma, Cat No. PKH67GL). The stained exosomes were put into a 100 kDa filter (Millipore, Cat No. UFC510008), centrifuged at 14000 rpm for 5 minutes, and 1X PBS was added to the supernatant to perform further centrifugation, this process was repeated twice. The final exosome product was filtered through a 0.2 µm filter with a 27G needle and added to treat SKOV3 cells, which are ovarian cancer cells expressing a folate receptor, followed by incubation for 24 hours. After completing the incubation, the cells were washed twice with 1X DPBS, the medium was exchanged with a SKOV3 medium, and a green fluorescence intensity was measured using Cytation 5 (Biotek).

**[Table 2]**

| **Recombinant plasmid** | **Detailed sequence** |
|---|---|
| GNSTM-LAMP2-ΔEC/IC-SKOV3 (SEQ ID NO: 35) | ATGGTGTGCTTCCGCCTCTTCCCGGTTCCGGGCTCAGGGCTCGTTCTGGTCT GCCTAGTCCTGGGAGCTGTGCGGTCTTATGCAGGTAACTCGACTATGGGCA GTGGAATGCATACCGCACCGGGATGGGGCTATAACCTGTCG GGCAGTGGATCTGGATCCGGTGGCTCGAGTCTAATCCCAATTATAGTTGGT GCTGGTCTTTCAGGCTTGATTATCGTTATAGTGATTGCTTACGTAATTGGCA GAGGTAGCGGTggaagcggatacccatacgatgtgccagattacgctAAGTCGACGGTACCGCG GGCCCGGGATCCACCGGTCGCCACCATGGTGAGCAAGGGCGAGGAGCTGT TCACCGGGGTGGTGCCCATCCTGGTCGAGCTGGACGGCGACGTAAACGGCC ACAAGTTCAGCGTGTCCGGCGAGGGCGAGGGCGATGCCACCTACGGCAAG CTGACCCTGAAGTTCATCTGCACCACCGGCAAGCTGCCCGTGCCCTGGCCC ACCCTCGTGACCACCCTGACCTACGGCGTGCAGTGCTTCAGCCGCTACCCC GACCACATGAAGCAGCACGACTTCTTCAAGTCCGCCATGCCCGAAGGCTAC GTCCAGGAGCGCACCATCTTCTTCAAGGACGACGGCAACTACAAGACCCGC GCCGAGGTGAAGTTCGAGGGCGACACCCTGGTGAACCGCATCGAGCTGAA GGGCATCGACTTCAAGGAGGACGGCAACATCCTGGGGCACAAGCTGGAGT ACAACTACAACAGCCACAACGTCTATATCATGGCCGACAAGCAGAAGAAC GGCATCAAGGTGAACTTCAAGATCCGCCACAACATCGAGGACGGCAGCGT GCAGCTCGCCGACCACTACCAGCAGAACACCCCCATCGGCGACGGCCCCGT GCTGCTGCCCGACAACCACTACCTGAGCACCCAGTCCGCCCTGAGCAAAGA CCCCAACGAGAAGCGCGATCACATGGTCCTGCTGGAGTTCGTGACCGCCGC CGGGATCACTCTCGGCATGGACGAGCTGTACAAGTAA |

### (2) Experimental results

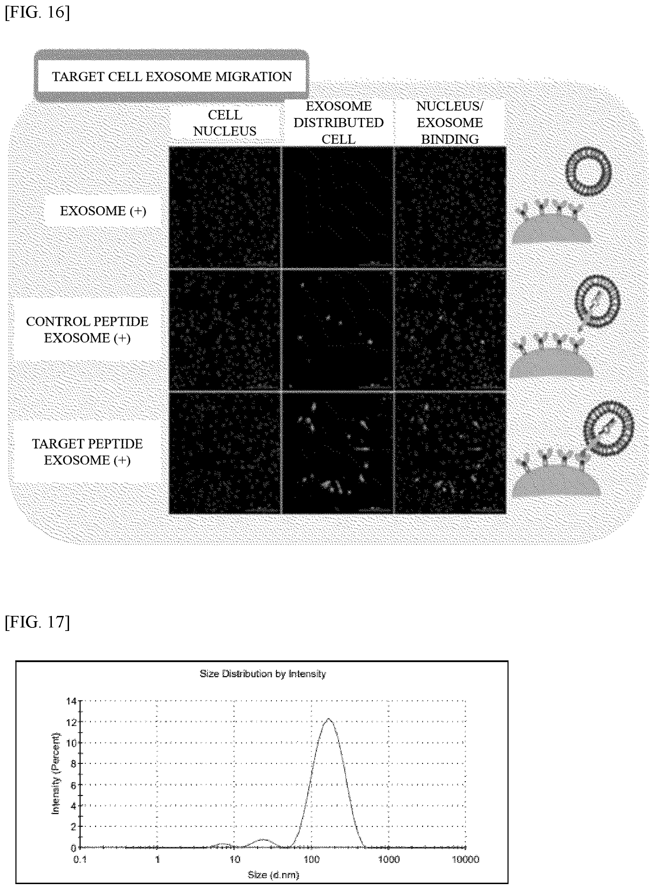

As a result of comparing the fluorescence expression of SKOV3 cells treated with each of exosomes that did not overexpress a plasmid (exosome (+)), exosomes in which FLAG in GNSTM-LAMP2-△EC/IC-GFP of FIG. 3 was inserted (control peptide exosome (+)), and exosomes in which a cancer-targeting peptide was inserted at the FLAG position in GNSTM-LAMP2-△EC/IC-GFP (target peptide exosome (+)), it was confirmed that the fluorescence expression of overexpressed exosomes was significantly stronger than those of the other two groups and a large amount of the exosomes were distributed (FIG. 16).

This shows that a plasmid carrying a sequence encoding a target peptide can be stably expressed in cells and exosomes, is active on the surface of the exosomes, and can selectively migrate in large quantities to cells expressing a cancer targeting peptide-binding receptor, suggesting the possibility of use as a therapeutic material based on cancer targeting through the effective expression of a biomolecule.

### 4. Measurement of exosome size distribution

### (1) Experimental method

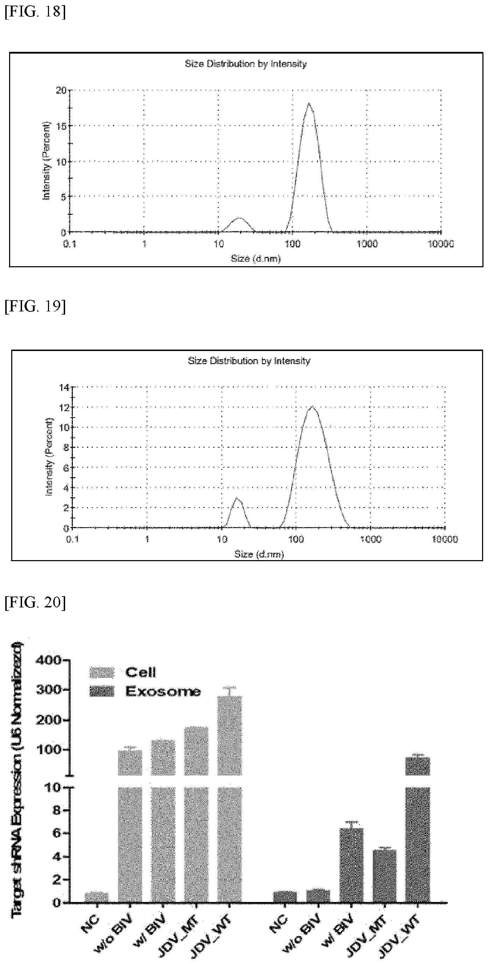

Exosomes of a cell line that was not transformed with a plasmid (FIG. 17), GNSTM-LAMP2-GFP (FIG. 18), and GNSTM-LAMP2-△EC/IC-GFP (FIG. 19) were overexpressed according to the method of Example 2-2 and cultured. After culture, the cells and the culture solution were collected in a 50 mL conical tube (SPL, Cat No. 50040) and centrifuged at 1000 rpm for 2 minutes, and the supernatant was further centrifuged at 4000 rpm for 30 minutes to remove cell debris. The final supernatant was added to a 10 kDa filter tube (Millipore, Cat No. UFC9010), and centrifuged at 4000 rpm for 30 minutes. Finally, the remaining supernatant was mixed with 1X PBS and further centrifuged, this process was repeated twice, and then the supernatant remaining in the filter tube was filtered through a 0.2 µm filter using a syringe with a 27-G needle, thereby obtaining exosomes of a single size. The size of the obtained exosomes was measured by dynamic light scattering (DLS).

### (2) Experimental results

As confirmed in FIGS. 17 to 19, there were no difference in size of exosomes of a cell line that was not transformed with a plasmid (FIG. 17), and exosomes overexpressing GNSTM-LAMP2-GFP (FIG. 18)- and GNSTM-LAMP2-△EC/IC-GFP (FIG. 19). Despite removing both the extracellular domain and intracellular domain of LAMP2, there was no difference in exosome size upon overexpression. This can be seen as meaning that there is no change in the basic properties of exosomes after overexpression even when LAMP2-△EC/IC of the present invention was used, suggesting that there is no problem in utilizing conventionally known exosomes for various biological purposes.

### Experimental Example 2. Stable expression and delivery of active RNA to target using modified peptide

### 1. Confirmation of expression level

### (1) Experimental method

After inserting a gene encoding a FLAG protein as a targeting peptide and a gene encoding an RNA-binding protein as an active protein into each of the plasmid framework structures of FIG. 5, each of the recombinant plasmids was overexpressed by the method of Example 2-2.

To confirm the expression level of active shRNA in cells, after completing culture by the method of Example 2-2, the cells and the culture solution were collected in a 50 mL conical tube (SPL, Cat No. 50040) and centrifuged at 1000 rpm for 2 minutes. Subsequently, the collected cells were washed twice with 1X PBS, RNA was isolated by a Trizol method (ThermoFisher Scientific, Cat No. 15596026), the poly A sequence was bound to 500 ng of RNA using a polyadenylation kit (Enzynomics, Cat No. EX041S), and cDNA was synthesized according to a gene specific primer cDNA synthesis method using primers specific for the introduced shRNA sequence of a PrimeScript^{™} cDNA kit (Takara,Cat No. 6210A). After cDNA synthesis, an shRNA expression level was measured by TOPreal^{™} Probe qPCR PreMix (Enzynomics, Cat. No. RT600S) using a target shRNA sequence Taqman probe.

To confirm the active shRNA expression level in exosomes, after completing culture by the method of Example 2-2, the cells and the culture solution were collected in a 50 mL conical tube (SPL, Cat No. 50040) and centrifuged at 1000 rpm for 2 minutes, and then the supernatant was added and centrifuged at 4000 rpm for 30 minutes to remove cell debris. The final supernatant was added to a 10 kDa filter tube (Millipore, Cat No. UFC9010) and centrifuged at 4000 rpm for 30 minutes. Finally, the remaining supernatant was mixed with 1X PBS and further centrifuged, this process was repeated twice, and then the supernatant remaining in the filter tube was filtered through a 0.2 µm filter using a syringe with a 27-G needle, thereby obtaining exosomes of a single size. After isolating RNA from the collected exosomes by a Trizol method (ThermoFisher Scientific, Cat No. 15596026), the poly A sequence was bound to 500 ng RNA using a polyadenylation kit (Enzynomics, Cat No. EX041S), and cDNA was synthesized according to a gene specific primer cDNA synthesis method using primers specific for the introduced shRNA sequence of a PrimeScript^{™} cDNA kit (Takara, Cat No. 6210A). After cDNA synthesis, an shRNA expression level was measured by TOPrealTM Probe qPCR PreMix (Enzynomics, Cat. No. RT600S) using a target shRNA sequence Taqman probe.

To measure a GFP expression level in SKOV3 cells treated with active shRNA-containing exosomes, after completing culture by the method of Example 2-2 regarding exosome isolation, the cells and the culture solution were collected in a 50 mL conical tube (SPL, Cat No. 50040) and centrifuged at 1000 rpm for 2 minutes, and then the supernatant was added and centrifuged at 4000 rpm for 30 minutes to remove cell debris. The final supernatant was added to a 10 kDa filter tube (Millipore, Cat No. UFC9010) and centrifuged at 4000 rpm for 30 minutes. Finally, the remaining supernatant was mixed with 1X PBS and further centrifuged, this process was repeated twice, and then the supernatant remaining in the filter tube was filtered through a 0.2 µm filter using a syringe with a 27-G needle, thereby obtaining exosomes of a single size.

To verify the expression suppression effect of shGFP-containing exosomes in GFP-overexpressing cells, 1×10⁵ SKOV3 cells were dispensed into each well of a 6-well plate and cultured for 24 hours. 500 ng of GFP fluorescence vector pAcGFP1-N1 (Clontech, PT3716) was introduced into each well according to the method of a PolyJet^{™} transfection kit (SignaGen^{®} Laboratories, Cat No. SL100688). After 24 hours, the medium was exchanged with a fresh medium, and 50 µg of the isolated shRNA-containing exosomes were added to each well. 48 hours after treatment, GFP fluorescence and a protein expression level were measured.

### (2) Experimental results

All models that overexpressed four plasmids such as GNSTM-LAMP2-△EC/IC-shGFP, GNSTM-LAMP2-△EC/IC-shGFP-BIV, GNSTM-LAMP2-△EC/IC-shGFP-JDV(WT), and GNSTM-LAMP2-△EC/IC-shGFP-JDV(MT) exhibited higher expression levels in cells, compared to an exosome only-treated group (NC) and a control. Particularly, when a BIV-derived RBP and a JDV-derived RBP were inserted as active proteins, compared to when no RBP was inserted (w/o BIV: RBP-free structure), the shGFP expression levels in all exosomes were significantly higher than those of the other groups. When the wild-type JDV-derived RBP was inserted, it was most effective with a difference of approximately 80 times compared to when the BIV-derived RBP was inserted (FIG. 20).

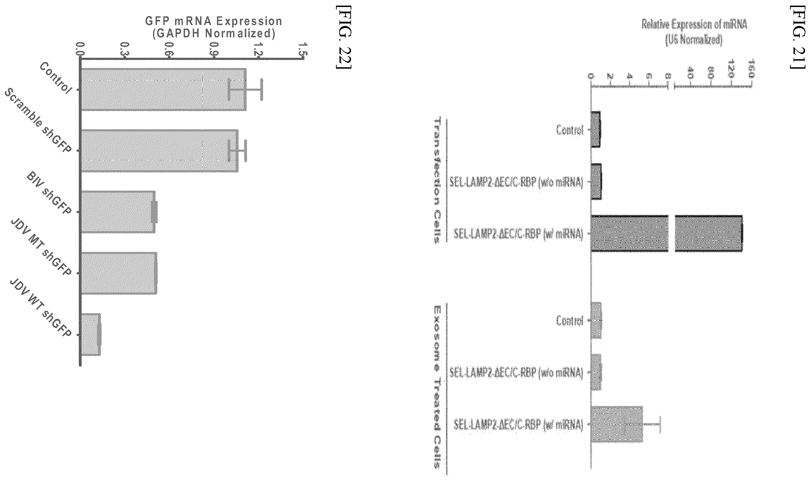

In addition, even when a SEL-LAMP2-△EC/IC-(pri-miRNA-199)-RBP plasmid, which is a glycosylation model using SEL rather than GNSTM, was overexpressed, it was confirmed that miRNA-199 was highly expressed in the transfected cells (FIG. 21).

These experimental results show that, due to the removal of both the extracellular domain and intracellular domain of LAMP2 and the introduction of glycosylation, RNA and the active protein can be stably expressed in all of the transfected cells and exosomes derived therefrom, ultimately suggesting the possibility of delivering a bioactive nucleic acid molecule through exosomes.

### 2. Confirmation of delivery level

When the SEL-LAMP2-△EC/IC-(pri-miRNA-199)-RBP plasmid, which is a glycosylation model using SEL, was overexpressed, it can be confirmed that miRNA-199 was excellently expressed even in cells treated with exosomes isolated after removing cells transfected by the plasmid (FIG. 21).

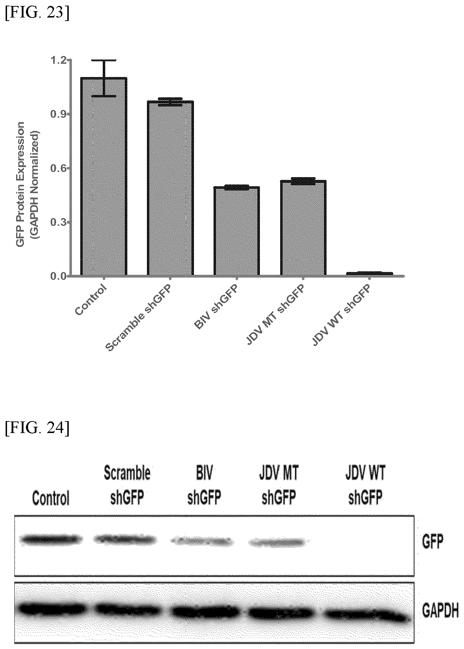

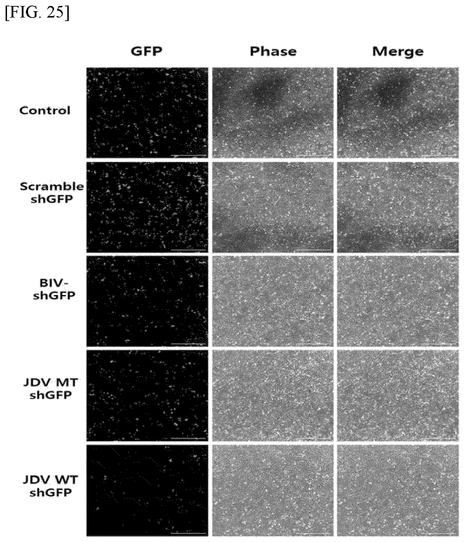

In addition, even when four glycosylation models using GNSTM, such as GNSTM-LAMP2-△EC/IC-shGFP-BIV, GNSTM-LAMP2-△EC/IC-shGFP-JDV(WT), GNSTM-LAMP2-△EC/IC-shGFP-JDV(MT) plasmids, were overexpressed, shGFP was also excellently expressed in GFP-transformed SKOV3 cell lines treated with exosomes isolated after removing cells transfected with the plasmids, confirming that the GFP expression (RNA level and protein level) was very effectively inhibited (FIGS. 22 and 23). Particularly, when a JDV(WT)-derived protein was used as an active protein as an RBP, an excellent effect was exhibited, and such tendency was the same even in the Western blotting results and fluorescence analysis results (FIGS. 24 and 25). The fluorescence analysis results showed that, particularly, when the GNSTM-LAMP2-△EC/IC-shGFP-JDV(WT) plasmid was overexpressed, GFP mRNA and protein can be suppressed up to 90 to 99%.

These experimental results mean that, due to the removal of both the extracellular domain and intracellular domain of LAMP2 and the introduction of glycosylation, RNA and an active protein were stably expressed in both the transfected cells and exosomes derived therefrom, and ultimately, the binding between RNA in the exosomes and an RBP was effectively dissociated, thereby successfully delivering the RNA to the target cells. Particularly, in the case of a plasmid into which the JDV-derived RBP and glycosylated LAMP2-△EC/IC were inserted, it is shown that the activity of shRNA can be maximized.

The scope of the present invention is defined by the appended claims and should be construed to encompass all modifications and alterations derived from meanings, the scope and equivalents of the appended claims.

## Claims

1. A plasmid platform for the stable expression and delivery of a biomolecule, comprising:
a nucleic acid sequence encoding a modified protein from which the intracellular domain or extracellular domain of lysosome-associated membrane glycoprotein 2B (LAMP-2B), or a combination thereof has been removed.

2. The plasmid platform of claim 1, wherein the plasmid platform comprises a nucleic acid sequence encoding a protein from which the intracellular domain of LAMP-2B has been removed.

3. The plasmid platform of claim 1, wherein the plasmid platform comprises a nucleic acid sequence encoding a protein from which the intracellular domain and extracellular domain of LAMP-2B have been removed.

4. The plasmid platform of claim 1, wherein the nucleic acid sequence has a nucleic acid sequence having 70% or more homology with a nucleic acid sequence of any one of SEQ ID NOs: 2 to 4.

5. The plasmid platform of claim 1, wherein the nucleic acid sequence has a nucleic acid sequence having 70% or more homology with a nucleic acid sequence of SEQ ID NO: 2 or 4.

6. The plasmid platform of claim 1, wherein the nucleic acid sequence has a nucleic acid sequence having 70% or more homology with a nucleic acid sequence of SEQ ID NO: 4.

7. The plasmid platform of claim 1, further comprising a nucleic acid sequence encoding a glycosylated region.

8. The plasmid platform of claim 7, wherein the nucleic acid sequence encoding the glycosylated region is located in an extracellular region direction based on the nucleic acid sequence encoding the modified protein.

9. The plasmid platform of claim 7, wherein the nucleic acid sequence encoding the glycosylated region includes a nucleic acid sequence having 70% or more homology with a nucleic acid sequence of any one of SEQ ID NOs: 11 to 13.

10. The plasmid platform of claim 7, wherein the nucleic acid sequence encoding the glycosylated region includes a nucleic acid sequence having 70% or more homology with a nucleic acid sequence of SEQ ID NO: 11.

11. The plasmid platform of claim 1, wherein the plasmid platform includes a nucleic acid sequence having 70% or more homology with a nucleic acid sequence of SEQ ID NO: 4 and a nucleic acid sequence having 70% or more homology with a nucleic acid sequence of SEQ ID NO: 11.

12. A recombinant plasmid for the stable expression and delivery of a biomolecule, further comprising a nucleic acid sequence encoding a biomolecule to be expressed and delivered in the plasmid platform of any one of claims 1 to 11.

13. The recombinant plasmid of claim 12, wherein the nucleic acid sequence encoding a biomolecule is located i) between the nucleic acid sequence encoding the glycosylated region and the nucleic acid sequence encoding the modified protein, ii) in an intracellular region direction based on the nucleic acid sequence encoding the modified protein, or iii) in both locations.

14. The recombinant plasmid of claim 12, wherein the biomolecule is at least one selected from the group consisting of a nucleic acid molecule, an aptamer, a peptide, a protein, a glycoprotein, a lipoprotein, an immunoglobulin, a hormone, a growth factor, a recombinase, and a fluorescent protein.

15. An exosome for the stable expression and delivery of a biomolecule, comprising a product expressed from the recombinant plasmid of claim 12.

16. The exosome of claim 15, wherein the biomolecule includes a material that is expressed outside the exosome and specifically binds to the surface of a target cell.

17. A composition for diagnosing cancer, comprising:
the exosome of claim 15,
wherein the biomolecule specifically binds to a protein specifically expressed on the surface of cancer cells.

18. The composition of claim 17, wherein the cancer is ovarian cancer.

19. A pharmaceutical composition for preventing or treating cancer, comprising:
the exosome of claim 15,
wherein the biomolecule specifically binds to a protein specifically expressed on the surface of cancer cells and includes a therapeutic material to be delivered into cancer cells.

20. The pharmaceutical composition of claim 17, wherein the cancer is ovarian cancer.
